# FASCICULE DE BREVET EUROPEEN

(11) **EP 2 318 440 B1**
(45) Date de publication et mention de la délivrance du brevet: **23.09.2015**
(21) Numéro de dépôt: 09784326.2
(22) Date de dépôt: 31.07.2009
(51) Int. Cl.: C07K 16/28, A61P 35/00, A61K 39/395, C07K 16/30

(54) **ANTICORPS SE LIANT AUX RECEPTEURS DE L'ADRENOMEDULLINE ET LEURS UTILISATIONS COMME MEDICAMENT**
AN ADRENOMEDULLINREZEPTOREN BINDENDE ANTIKÖRPER UND DEREN VERWENDUNG ALS ARZNEIMITTEL
ANTIBODIES BINDING TO ADRENOMEDULLIN RECEPTORS AND USES THEREOF AS DRUGS

(30) Priorité: 31.07.2008 FR 0804382
(43) Date de publication de la demande: 11.05.2011
(73) Titulaire: Université d'Aix-Marseille, 13284 Marseille Cedex 07 (FR); Assistance Publique Hôpitaux de Marseille, 13354 Marseille (FR)
(72) Inventeur: OUAFIK, L'Houcine, F-13005 Marseille (FR); MABROUK, Kamel, F-13170 Les Pennes Mirabeau (FR); KAAFARANY, Itidal, 47200 Marmande (FR); MARTIN, Pierre-Marie, F-13008 Marseille (FR)
(74) Mandataire: Rançon, Xavier Lucien Abel
(86) Numéro de dépôt international: PCT/FR2009/000964
(87) Numéro de publication internationale: WO 2010/012911

(56) Documents cités:
- WO-A-2007/045927
- FERNANDEZ-SAUZE SAMANTHA ET AL: "Effects of adrenomedullin on endothelial cells in the multistep process of angiogenesis: involvement of CRLR/RAMP2 and CRLR/RAMP3 receptors." INTERNATIONAL JOURNAL OF CANCER. JOURNAL INTERNATIONAL DU CANCER 1 MAR 2004, vol. 108, no. 6, 1 mars 2004 (2004-03-01), pages 797-804, XP002520101 ISSN: 0020-7136 cité dans la demande
- ABASOLO I ET AL: "Adrenomedullin inhibits prostate cancer cell proliferation through a cAMP-independent autocrine mechanism" BIOCHEMICAL AND BIOPHYSICAL RESEARCH COMMUNICATIONS, ACADEMIC PRESS INC. ORLANDO, FL, US, vol. 322, no. 3, 24 septembre 2004 (2004-09-24), pages 878-886, XP004536468 ISSN: 0006-291X
- ISHIKAWA T ET AL: "Adrenomedullin antagonist suppresses in vivo growth of human pancreatic cancer cells in SCID mice by suppressing angiogenesis" ONCOGENE, NATURE PUBLISHING GROUP, GB BASINGSTOKE, HANTS, vol. 22, no. 8, 1 février 2003 (2003-02-01), pages 1238-1242, XP002967674 ISSN: 0950-9232
- NAKAMURA MISA ET AL: "Adrenomedullin: a tumor progression factor via angiogenic control" CURRENT CANCER DRUG TARGETS, BENTHAM SCIENCE PUBLISHERS, HILVERSUM, NL, vol. 6, no. 7, 1 novembre 2006 (2006-11-01), pages 635-643, XP009096862 ISSN: 1568-0096
- KAAFARANI ITIDAL ET AL: "Targeting adrenomedullin receptors with systemic delivery of neutralizing antibodies inhibits tumor angiogenesis and suppresses growth of human tumor xenografts in mice.", THE FASEB JOURNAL : OFFICIAL PUBLICATION OF THE FEDERATION OF AMERICAN SOCIETIES FOR EXPERIMENTAL BIOLOGY OCT 2009 LNKD- PUBMED:19546305, vol. 23, no. 10, October 2009 (2009-10), pages 3424-3435, ISSN: 1530-6860

## Description

La présente invention concerne le domaine des anticorps utilisés comme médicament, destiné notamment au traitement des tumeurs. Plus particulièrement, l'invention concerne des anticorps se liant aux protéines formant les récepteurs de l'adrénomédulline et leurs utilisations comme médicament.

L'angiogenèse est le processus fondamental par lequel de nouveaux vaisseaux sanguins sont formés. Le processus comporte la migration des cellules endothéliales vasculaires dans un tissu suivi de l'organisation desdites cellules endothéliales en vaisseaux.

L'angiogenèse joue un rôle déterminant dans la croissance tumorale et le développement des métastases. Dans les tissus sains, il existe un équilibre entre facteurs pro-angiogéniques et facteurs anti-angiogéniques ; ces derniers étant exprimés ou dérégulés dans les processus tumoraux (HANAHAN and FOLKMAN, Cell, 1996, 86:353-364). Au-delà d'un certain volume tumoral, la croissance de la tumeur nécessite le développement d'une néovascularisation qui lui apportera l'oxygène et les nutriments nécessaires. Les cellules tumorales elles-mêmes sécrètent des facteurs angiogéniques et stimulent leur microenvironnement pour augmenter la biodisponibilité des facteurs nécessaires au développement de l'angiogenèse tumorale.

L'existence d'un réseau vasculaire très développé au sein des tumeurs est connue depuis de nombreuses années. Dès 1971, FOLKMAN (N Engl J Med., 1971, 285:1182-6) a émis l'hypothèse que la croissance tumorale était dépendante de la néovascularisation (angiogenèse) et que le passage de la phase latente à la phase agressive était contrôlé directement par la néovascularisation grâce à des substances diffusibles provenant de la tumeur.

Le contrôle de l'angiogenèse implique plusieurs facteurs. Elle est déclenchée par un déséquilibre de la balance entre les facteurs pro-angiogéniques (par exemple, facteur de croissance endothéliale vasculaire (Vascular endothelial Growth Factor : VEGF), facteur de croissance des fibroblastes (Fibroblast Growth Factor : FGF), facteur de croissance épidermal (Epidermal Growth Factor : EGF), facteur de croissance dérivé des plaquettes (Platelet Derived Growth Factor : PDGF), angiopoïétines, ou encore angiogénine) et les facteurs anti-angiogéniques (par exemple, endostatine et angiostatine, thrombospondines, vasostatine, prolactine, ou encore interférons).

Les cellules tumorales mais aussi les cellules inflammatoires, macrophages, lymphocytes, et myofibroblastes présentes dans le micro-environnement tumoral sécrètent des facteurs angiogéniques.

Il est classique de distinguer deux phases au cours de l'angiogenèse. La première phase est une phase d'induction qui implique la déstabilisation de la vascularisation tissulaire préexistante et la destruction de la membrane basale entourant les vaisseaux préexistants, et qui nécessite la prolifération et la migration des cellules endothéliales, ainsi que leur différenciation en structures capillaires. La seconde phase est une phase de stabilisation/maturation au cours de laquelle des cellules périvasculaires (péricytes) sont recrutées, aboutissant à la stabilisation des néo-capillaires, et au cours de laquelle une membrane basale est reconstituée (HANAHAN and FOLKMAN, 1996, cité ci-dessus).

Il est maintenant bien établi que la croissance d'une tumeur et la formation de métastases sont directement dépendantes de l'angiogenèse suggérant que l'inhibition de l'angiogenèse peut représenter une approche efficace pour empêcher la progression tumorale et contrôler la diffusion métastatique.

L'adrénomédulline (AM), isolée à partir de phéochromocytome humain (cancer de la médullosurrénale), est un peptide vasoactif qui agit localement comme une hormone autocrine/paracrine et exerce de multiples actions biologiques (HINSON et al., Endocr Rev., 2000, 21:138-67 ; CARON and SMITHIES, Proc Natl Acad Sci USA, 2001, 98:615-619 ; SHINDO et al., Circulation, 2001, 104:1964-1971).

Plusieurs études ont montré que des sites de liaison de l'adrénomédulline sont présents dans les cellules de la plupart des tissus, tels que le coeur, le rein, le cerveau, le poumon et la glande surrénale. Des sites de liaison sont également présents dans les cellules du stroma des tumeurs. Il a été aussi mis en évidence un rôle de l'adrénomédulline dans la croissance tumorale (OUAFIK et al., Am J Pathol., 2002, 160:1279-92 ; MARTINEZ et al., J Natl Cancer Inst., 2002, 94:1226-37 ; OEHLER et al., Oncogene, 2001, 20:2937-45 ; ISHIKAWA et al., Oncogene, 2003, 22:1238-1242).

Ce peptide peut aussi jouer un rôle positif dans la régulation de l'angiogenèse lors du remodelage vasculaire en réponse à l'ischémie, dans l'appareil reproducteur de la femme, durant le développement vasculaire embryonnaire, et lors du développement et de la vascularisation du placenta.

Récemment, plusieurs équipes ont mis en évidence un rôle de l'adrénomédulline sur la prolifération, la migration et l'invasion des cellules endothéliales (OUAFIK *et al.,* 2002, cité ci-dessus ; KIM et al., FASEB J., 2003, 17:1937-9; FERNANDEZ-SAUZE et al., Int J Cancer, 2004, 108:797-804):

Par des tests *d'angiogenèse in vivo* et *in vitro,* il a été montré que l'adrénomédulline agit sur une des dernières étapes de la néovascularisation qui consiste en la réorganisation des cellules endothéliales en tubules et ce, indépendamment du VEGF (FERNANDEZ-SAUZE *et al.,* 2004, cité ci-dessus).

Plusieurs études démontrent que l'adrénomédulline possède des propriétés angiogéniques dans la plupart des tumeurs (sein, prostate, colon, poumon, rein, voies respiratoires, vessie) (OUAFIK *et al.,* 2002, cité ci-dessus; FERNANDEZ-SAUZE *et al.,* 2004, cité ci-dessus ; NIKITENKO et al., Br J Cancer, 2006, 94:1-7). Chez des souris hétérozygotes adrénomédulline +/-, le volume tumoral diminue par rapport aux souris sauvages (IIMURO et al., Circ. Res., 2004, 95:415-423). Cet effet est associé à une réduction de la néo-vascularisation. Le blocage de l'action de l'adrénomédulline par un antagoniste (adrénomédulline₂₂₋₅₂) inhibe la croissance de tumeurs pancréatiques xénogreffées en déstabilisant la vascularisation des tumeurs (ISHIKAWA et al., Oncogene, 2003, 22:1238-1242). Des effets similaires ont été observés dans des xénogreffes développées à partir des cellules gliales tumorales (OUAFIK *et al.,* 2002, cité ci-dessus).

La découverte de la protéine sérique AMBP-1 (Adrenomedullin Binding Protein-1) liant l'adrénomédulline laisse supposer une régulation de la biodisponibilité de cette dernière. L'AMBP-1 a été décrite et caractérisée comme étant le facteur H du complément humain. De manière générale, les protéines de liaison limitent le transport du peptide dans l'espace interstitiel et l'accès à ses récepteurs spécifiques. Elles modulent aussi l'activité biologique du peptide et le protègent contre la clairance métabolique par les protéases, prolongeant ainsi sa demi-vie dans la circulation.

Les récepteurs de l'adrénomédulline (AMR) sont des complexes multiprotéiques composés de l'association d'au moins deux protéines, le CRLR (Calcitonin Receptor Like Receptor) et une protéine RAMP (Receptor Activity-Modifying Protein) (McLATCHIE et al., Nature, 1998, 6683:333-9)

Le récepteur CRLR a été isolé en 1993 (NJUKI et al., Clin Sci., 1993 4:385-8; CHANG et al., Neuron., 1993, 6:1187-95). Il comporte sept domaines transmembranaires couplés à une protéine G. La séquence du CRLR fut établie chez l'Homme en 1996 (AIYAR et al., J Biol Chem., 1996, 19:11325-9) et chez le porc en 1998 (ELSHOURBAGY et al., Endocrinology, 1998, 4:1678-83). Le CRLR appartient à la classe II des RCPG (« *G-protein-coupled receptors* »), classe qui regroupe des récepteurs de peptides tels que le glucagon et les peptides apparentés au glucagon (GLP), la sécrétine, la parathormone ou la calcitonine. Les RCPG sont de nature polypeptidique et comportent une partie extracellulaire portant le site de liaison du ligand, une partie transmembranaire à sept hélices et une partie intracellulaire en contact avec les protéines G qui assurent le transfert et l'amplification du signal reçu par le récepteur. On peut observer trois boucles extracellulaires (nommées E1, E2 et E3) et trois boucles intracellulaires (I1, 12 et 13) (BOCKAERT and PIN, Embo J., 1999, 18:1723-1729). Ces protéines peuvent être sujettes à des modifications post-traductionnelles, de type N-glycosylation, acétylation par des composés lipidiques formant parfois une pseudo-quatrième boucle intracellulaire (I4) (ASSIE et al., EMC-Endocrinologie, 2004, 1:169-199).

Pendant quelques années, une certaine confusion a régné quant à la nature exacte du récepteur de l'adrénomédulline du fait de l'homologie de l'adrénomédulline avec le CGRP (Calcitonine Gene Related Peptide) et de son appartenance à la famille des peptides Calcitonine/CGRP/Amyline. En 1998, McLATCHIE et ses collaborateurs (référence citée ci-dessus) ont démontré que le récepteur CRLR peut générer deux récepteurs pharmacologiquement distincts par association à une famille de protéines, de 160 acides aminés (14-21 KDa), à un seul domaine transmembranaire, nommées RAMPs. Le CRLR n'est correctement fonctionnel qu'à l'état de dimère avec une protéine RAMP.

Il existe trois isoformes protéiques de RAMPs : RAMP1, 2 et 3. Ces protéines ont moins de 30% d'identité de séquence entre elles, mais présentent des similitudes d'organisation structurale. Chez l'Homme, les gènes codant RAMP1, RAMP2 et RAMP3 sont portés respectivement par les chromosomes 2, 17 et 7. Les protéines RAMPs sont constituées d'un seul domaine transmembranaire ; l'extrémité N-terminale extracellulaire est relativement longue et joue un rôle important dans la spécialisation et la fonctionnalité du récepteur (CGRP ou adrénomédulline) (KUWASAKO et al., J Biol Chem., 2001, 275:29602-9).

Deux fonctions essentielles sont attribuées aux protéines RAMPs : la détermination du récepteur et le transport intra-cellulaire.
- Détermination du récepteur : le rôle fondamental des protéines RAMPs est de définir la spécificité du ligand qui interagit directement à la surface cellulaire. RAMP1 présente le CRLR comme une glycoprotéine mature pour former le récepteur du CGRP. De même RAMP2 et RAMP3 présentent le CRLR comme une glycoprotéine mature pour former les récepteurs de l'adrénomédulline. Ainsi la nature des protéines RAMPs présentes dans un type cellulaire, les interactions protéiques qui s'établissent entre les différents partenaires (CRLR, RAMP1, RAMP2, RAMP3) et la proportion de chacune des protéines permettent aux cellules de répondre spécifiquement à différents neuropeptides (BÜHLMANN et al., FEBS Lett., 2000, 486:320-4 ; CHAKRAVARTY et al., Br J Pharmacol., 2000, 130:189-95).
- Transport intracellulaire : le CRLR nécessite la co-expression des protéines RAMPs pour son transport vers la membrane cytoplasmique (SEXTON et al., Cell Signal, 2001, 13:73-83). La réciproque est vraie : les protéines RAMPs ont besoin du CRLR pour leur translocation à la surface cellulaire (FLAHAUT et al., J Biol Chem., 2002, 277:14731-7).

La croissance des tumeurs solides est contrôlée par des mécanismes intratumoraux et par des interactions entre la tumeur et le tissu environnant. En phase de quiescence, peu de vaisseaux sont décelés. En revanche, lors de la phase de croissance et lors de la phase invasive, l'angiogenèse est massive. Il existe une étroite corrélation entre croissance tumorale et le nombre de capillaires intra-tumoraux. Ainsi les tumeurs solides dépendantes de l'angiogenèse présentent une phase pré-angiogénique latente, et une phase angiogénique agressive.

Le traitement des tumeurs, particulièrement des tumeurs solides repose principalement sur la chirurgie, la radiothérapie et la chimiothérapie. Cependant, malgré les progrès obtenus dans ces domaines et les résultats encourageants, il s'avère crucial de disposer de nouveaux anticancéreux à mécanisme d'action différent des anticancéreux disponibles, entre autre dans le domaine des thérapeutiques ciblées, pour augmenter l'efficacité du traitement notamment dans les cas d'apparition de résistance à un traitement et/ou d'effets indésirables trop importants.

A côté des thérapies visant la destruction des cellules prolifératives (chimiothérapie) et l'hormonothérapie dans le cadre de cancers hormono-dépendants (sein, prostate), les thérapeutiques ciblées visent l'ensemble des voies qui contribuent au développement tumoral, comme les signaux de prolifération, le cycle cellulaire, l'apoptose, l'invasion ou encore l'angiogenèse (FOLKMAN, Nat Rev Drug Discov., 2007, 6:273-286; NERI and BICKNELL, Nat Rev Cancer, 2005, 5:436-446).

La présence d'une néoangiogenèse tumorale associée à la surexpression des ARNm de facteurs angiogéniques comme le VEGF et le FGF-2, ont abouti au développement d'inhibiteurs (anticorps spécifiques, oligonucléotides anti-sens, inhibiteurs pharmacologiques) par plusieurs sociétés pharmaceutiques. Un certain nombre de molécules, actuellement en essai clinique, interviennent dans des protocoles thérapeutiques conjointement aux traitements classiques.

On sait cependant que l'utilisation d'anticorps anti-VEGF pour le traitement des tumeurs, même si elle donne de bons résultats (arrêt rapide de la croissance de la tumeur), a des effets toxiques importants. En outre, une récidive (reprise de la croissance de la tumeur) peut intervenir à plus ou moins court terme après l'arrêt du traitement. De plus ces traitements visent les cellules endothéliales mais pas l'ensemble des cellules du stroma tumoral impliquées dans l'établissement d'une néoangiogenèse fonctionnelle.

Il demeure donc toujours un besoin en traitements efficaces visant à bloquer, voire inhiber, la croissance tumorale, et/ou à faire régresser la taille des tumeurs.

Dans ce contexte, le ciblage de l'adrénomédulline *via* ses récepteurs à des fins thérapeutiques, constitue une approche pertinente en raison de son mécanisme d'action qui porte à la fois sur les cellules endothéliales mais aussi, à la différence du VEGF, sur la cellule tumorale et sur l'ensemble des cellules du stroma, particulièrement les péricytes.

Ainsi, FERNANDEZ-SAUZE *et al.* (2004, cité ci-dessus) ont montré qu'*in vitro,* des mélanges d'anticorps polyclonaux anti-CRLR/anti-RAMP2 d'une part et anti-CRLR/anti-RAMP3 d'autre part inhibent la liaison spécifique de l'adrénomédulline à son récepteur sur plusieurs types cellulaires et bloquent la formation de tubes vasculaires. Plus récemment, il a été décrit dans la Demande Internationale WO 2007/045927 que des compositions pharmaceutiques comprenant des anticorps qui se lient spécifiquement aux protéines humaines RAMP2 ou RAMP3, peuvent être utiles pour traiter ou prévenir le cancer, *via,* par exemple, l'inhibition de l'angiogenèse ou de la prolifération des cellules cancéreuses.

Les Inventeurs ont préparé des anticorps se liant aux protéines qui forment les récepteurs de l'adrénomédulline et ont montré de manière surprenante, qu'un mélange d'au moins trois anticorps se liant à trois protéines différentes formant les récepteurs de l'adrénomédulline, plus particulièrement les protéines hCRLR, hRAMP2 et hRAMP3, présentait une efficacité anti-tumorale *in vitro* et/ou *in vivo* significativement plus importante par rapport à l'utilisation d'un seul anticorps anti-CRLR, anti-RAMP2 ou anti-RAMP3, ou même par rapport à l'utilisation d'un mélange de deux anticorps anti-CRLR/anti-RAMP2 ou anti-CRLR/anti-RAMP3.

La présente invention a donc pour objet, un mélange d'au moins trois anticorps et/ou fragments desdits anticorps se liant à trois des protéines formant les récepteurs de l'adrénomédulline, chaque anticorps et/ou fragments d'anticorps se liant à une protéine différente, et chaque anticorps et/ou fragments d'anticorps étant un antagoniste d'un desdits récepteurs de l'adrénomédulline, pour utilisation comme médicament.

On entend par « protéines formant les récepteurs de l'adrénomédulline », les protéines dont l'association forme les récepteurs de l'adrénomédulline (HINSON *et al.,* 2000, cité ci-dessus; McLATCHIE *et al.,* 1998, cité ci-dessus). De préférence, lesdites protéines sont des protéines de mammifère, et de préférence encore des protéines d'origine humaine. A titre d'exemples non limitatifs de protéines formant les récepteurs de l'adrénomédulline, on peut citer les protéines suivantes : la protéine CRLR *(calcitonin receptor like receptor*)*,* les protéines associées RAMPs (*receptor activity-modifying protein*), telles que les protéines RAMP2 et -3. Les séquences d'acides aminés des protéines CRLR, RAMP2 et -3 d'origine humaine sont respectivement disponibles sous les numéros d'accès gi|5031621, gi|118572585 et gi|5032023 dans la base de données GENBANK.

Selon un mode de mise en oeuvre préféré de la présente invention, les trois protéines formant les récepteurs de l'adrénomédulline sont la protéine CRLR et les protéines RAMP2 et 3.

Selon un autre mode de mise en oeuvre avantageux de la présente invention, lesdits anticorps et fragments d'anticorps se lient à un domaine extracellulaire des protéines formant les récepteurs de l'adrénomédulline concernées, et plus particulièrement aux peptides de séquences SEQ ID NO: 1 ou 2 pour les anticorps anti-CRLR, aux peptides de séquences SEQ ID NO: 3 ou 4 pour les anticorps anti-RAMP2 et aux peptides de séquences SEQ ID NO: 5 ou 6 pour les anticorps anti-RAMP3.

L'invention englobe les anticorps polyclonaux ou monoclonaux, naturels, recombinants ou synthétiques, les anticorps chimériques tels que les anticorps humanisés, ainsi que leurs fragments (par exemple : Fab, Fv, scFv) ayant conservé leur capacité de liaison auxdites protéines, ou plus particulièrement aux peptides de séquences SEQ ID NO: 1 ou 2 pour les anticorps anti-CRLR, aux peptides de séquences SEQ ID NO: 3 ou 4 pour les anticorps anti-RAMP2 et aux peptides de séquences SEQ ID NO: 5 ou 6 pour les anticorps anti-RAMP3.

Lesdits anticorps ou fragments d'anticorps sont des antagonistes des récepteurs de l'adrénomédulline, c'est-à-dire qu'ils bloquent (ou inhibent) d'une façon dose-dépendante la liaison de l'adrénomédulline à ces récepteurs.

Par « anticorps recombinant », on entend un anticorps produit par génie génétique (e.g., clonage, amplification).

Par « anticorps synthétique », on entend un anticorps produit par synthèse enzymatique et/ou chimique.

Les anticorps selon la présente invention sont susceptibles d'être obtenus par immunisation d'un animal avec une protéine formant les récepteurs de l'adrénomédulline, un peptide comprenant ou constitué d'un fragment d'au moins 20, de préférence 22 acides aminés de ladite protéine, ou un peptide comprenant ou constitué d'un peptide dérivé dudit fragment.

On entend par « peptide dérivé d'un fragment d'une protéine formant les récepteurs de l'adrénomédulline », un fragment de ladite protéine dans lequel un ou plusieurs résidus d'acides aminés a(ont) été délété(s), et/ou un ou plusieurs résidus d'acides aminés a(ont) été substitué(s) par un résidu d'acide aminé naturel ou non naturel ou de configuration de type D ou beta, et/ou un ou plusieurs résidus d'acides aminés naturel(s) ou non-naturel(s) y a(ont) été inséré(s), et/ou une ou plusieurs liaisons amides a(ont) été modifiée(s), étant entendu que ledit peptide dérivé d'un fragment d'une protéine formant les récepteurs de l'adrénomédulline conserve sa capacité à induire la production, par ledit animal, d'anticorps se liant à ladite protéine.

De manière avantageuse, chacun des peptides suivants peut être utilisé pour immuniser un animal afin d'obtenir un anticorps selon la présente invention :
- peptides dérivés respectivement des fragments S27-K51 et P89-R119 de la protéine hCRLR :
   SPEDSIQLGVTRNKIMTAQYEAYQK (SEQ ID NO:1),
   PDYFQDFDPSEKVTKIADQDGNWFRHPASNR (SEQ ID NO : 2),
- peptides dérivés respectivement des fragments K59-K81 et R91-R118 de la protéine hRAMP2 :
   KNYETAVQFAWNHYKDQMDPIEK (SEQ ID NO : 3),
   RPYSTLRDALEHFAELFDLGFPNPLAER (SEQ ID NO : 4),
- peptides dérivés respectivement des fragments L34-K55 et G91-E112 de la protéine hRAMP3 :
   LERLPLAGKAFADMMGKVDVWK (SEQ ID NO : 5),
   GFITGIHRQFFSNATVDRVHLE (SEQ ID NO : 6).

Le résidu d'acide aminé noté « A » correspond à un résidu alanine obtenu par substitution d'un résidu cystéine présent dans la séquence peptidique des protéines hCRLR, hRAMP2 ou hRAMP3 naturelles. Le fait de substituer le résidu cystéine par une alanine permet d'obtenir une séquence peptidique linéaire bien caractérisée et évite d'obtenir des mélanges de peptides comprenant des dimères (par formation de ponts disulfures interchaînes).

De préférence, l'animal immunisé est un mammifère, tel que par exemple le cheval, la chèvre, le lapin, le rat et la souris, et de préférence encore le lapin ou la souris.

Selon un autre mode de mise en oeuvre particulier de la présente invention, lesdits anticorps sont des anticorps polyclonaux, de préférence des anticorps polyclonaux de lapin.

Il est possible que le mélange d'anticorps et/ou fragments d'anticorps selon la présente invention comprenne à la fois des anticorps polyclonaux et des anticorps monoclonaux tels que définis ci-dessus.

Dans ledit mélange, lesdits anticorps et/ou fragments d'anticorps peuvent être présents en tout rapport de l'un vers les autres, comme par exemple un rapport compris entre 0,1 et 10. Un rapport préférentiel est un rapport de 1.

Avantageusement, ledit médicament est destiné au traitement préventif ou curatif de tumeurs, préférentiellement celles dont la vascularisation est nécessaire à leur croissance, plus particulièrement aux tumeurs solides.

Par « tumeurs solides » on entend par exemple les tumeurs du système nerveux central comme les gliomes (par exemple les glioblastomes), les tumeurs de la prostate, du foie, des os, du poumon, du colon, de la peau ou encore du rein.

L'invention englobe également les tumeurs à croissance rapide ainsi que les tumeurs en phase d'échappement thérapeutique.

L'utilisation comme médicament des anticorps et/ou fragments d'anticorps selon la présente invention peut être simultanée, séparée ou étalée dans le temps, notamment lors d'un traitement chez un sujet atteint d'un cancer.

La présente invention a aussi pour objet un mélange d'au moins trois anticorps et/ou fragments d'anticorps tels que définis ci-dessus, pour la préparation d'un médicament destiné au traitement des tumeurs, préférentiellement des tumeurs solides.

La présente invention a également pour objet une composition pharmaceutique comprenant au moins trois anticorps et/ou fragments d'anticorps tels que définis ci-dessus, et au moins un véhicule pharmaceutiquement acceptable.

A titre d'exemples non limitatifs de véhicule pharmaceutiquement acceptable, on peut citer les dispersants, solubilisants, stabilisants, conservateurs, etc. Des véhicules pharmaceutiquement acceptables utilisables dans des formulations (liquides et/ou injectables et/ou solides) sont notamment la méthylcellulose, l'hydroxyméthylcellulose, la carboxyméthylcellulose, les cyclodextrines, le polysorbate 80, le mannitol, la gélatine, le lactose, des huiles végétales ou animales, l'acacia, etc.

Ledit médicament ou ladite composition pharmaceutique peut se présenter sous la forme d'une solution saline, physiologique, isotonique et tamponnée, compatible avec un usage pharmaceutique et connu de l'Homme du métier.

Ledit médicament ou ladite composition pharmaceutique peut être formulé sous toute forme pharmaceutiquement acceptable, comme par exemple sous la forme de suspension injectable, de gels, huiles, comprimés, suppositoires, gélules, capsules, etc., éventuellement utilisé au moyen de formes galéniques ou de dispositifs assurant une libération prolongée et/ou retardée. Pour ce type de formulation, on utilise avantageusement un agent tel que la cellulose, des carbonates ou des amidons.

La quantité d'anticorps et/ou fragment d'anticorps utilisés à titre de médicament selon l'invention ou présente dans la composition pharmaceutique selon l'invention peut être modulée de façon à obtenir un taux circulant de principe actif (dans un liquide physiologique tel que le sang) nécessaire à l'obtention de l'effet thérapeutique désiré pour un sujet particulier. La quantité choisie dépendra de multiples facteurs, en particulier de la voie d'administration, de la durée d'administration, du moment de l'administration, de la vitesse d'élimination du composé, du ou des différents produits utilisés en combinaison avec ledit médicament ou ladite composition pharmaceutique, de l'âge, du poids et de la condition physique du patient, ainsi que de son histoire médicale, et de toutes autres informations connues en médecine.

La prescription du médecin traitant pourra commencer à des doses inférieures à celles généralement utilisées pour des anticorps, puis ces doses seront progressivement augmentées afin de mieux maîtriser l'apparition d'éventuels effets secondaires.

En général la dose journalière du composé sera la dose minimum pour obtenir l'effet thérapeutique. Cette dose dépendra des différents facteurs cités auparavant. Les doses seront en général comprises entre 0,1 et 100 mg par kg par jour pour l'Homme, et préférentiellement entre 4 et 25 mg par kg et par jour et encore plus avantageusement entre 7 et 14 mg par kg et par jour.

Si nécessaire, la dose journalière peut être administrée en deux, trois, quatre, cinq, six ou plus prises par jour ou par sous-doses multiples administrées par intervalles appropriés pendant la journée.

De manière avantageuse, la composition selon la présente invention est administrée de préférence par voie parentérale ou directement, si cela est possible dans la tumeur (administration intratumorale).

Le médicament ou la composition pharmaceutique selon la présente invention peut être utilisé seul ou en association avec au moins un autre composé thérapeutiquement actif, tel que par exemple un autre composé anticancéreux. L'utilisation dudit médicament ou de ladite composition pharmaceutique, et dudit composé thérapeutiquement actif peut être simultanée, séparée ou étalée dans le temps, notamment lors d'un traitement d'un sujet atteint d'un cancer.

La présente invention a aussi pour objet un anticorps, de préférence un anticorps polyclonal, se liant au domaine extracellulaire de la protéine CRLR, susceptible d'être obtenu par immunisation d'un animal non-humain, de préférence un lapin, avec un peptide choisi parmi les peptides de séquence SEQ ID NO : 1 et SEQ ID NO : 2, et son utilisation pour détecter *in vitro, ex vivo* ou *in vivo,* chez un animal, de préférence un mammifère, de préférence encore chez l'Homme, la protéine CRLR.

La présente invention a aussi pour objet un anticorps, de préférence un anticorps polyclonal, se liant au domaine extracellulaire de la protéine RAMP2, susceptible d'être obtenu par immunisation d'un animal non-humain, dé préférence un lapin, avec un peptide choisi parmi les peptides de séquence SEQ ID NO : 3 et SEQ ID NO : 4, et son utilisation pour détecter *in vitro, ex vivo* ou *in vivo,* chez un animal, de préférence un mammifère, de préférence encore chez l'Homme, la protéine RAMP2.

La présente invention a aussi pour objet un anticorps, de préférence un anticorps polyclonal, se liant au domaine extracéllulaire de la protéine RAMP3, susceptible d'être obtenu par immunisation d'un animal non-humain, de préférence un lapin, avec un peptide choisi parmi les peptides de séquence SEQ ID NO :5 et SEQ ID NO : 6, et son utilisation pour détecter *in vitro, ex vivo* ou *in vivo,* chez un animal, de préférence un mammifère, de préférence encore chez l'Homme, la protéine RAMP3.

La présente invention a aussi pour objet un procédé d'obtention d'un anticorps se liant à un domaine extracellulaire de CRLR, caractérisé en ce qu'il comprend une étape d'immunisation d'un animal non-humain avec le peptide de séquence SEQ ID NO : 2.

Les inventeurs décrivent également un procédé d'obtention d'un anticorps se liant à un domaine extracellulaire de CRLR, caractérisé en ce qu'il comprend une étape d'immunisation d'un animal avec un peptide choisi parmi les peptides de séquence SEQ ID NO : 1 et SEQ ID NO : 2.

La présente invention a aussi pour objet un procédé d'obtention d'un anticorps se liant au domaine extracellulaire de RAMP2, caractérisé en ce qu'il comprend une étape d'immunisation d'un animal non-humain avec le peptide de séquence SEQ ID NO : 4.

Les inventeurs décrivent également un procédé d'obtention d'un anticorps se liant au domaine extracellulaire de RAMP2, caractérisé en ce qu'il comprend une étape d'immunisation d'un animal avec un peptide choisi parmi les peptides de séquence SEQ ID NO : 3 et SEQ ID NO : 4.

La présente invention a aussi pour objet un procédé d'obtention d'un anticorps se liant au domaine extracellulaire de RAMP3, caractérisé en ce qu'il comprend une étape d'immunisation d'un animal non-humain avec le peptide de séquence SEQ ID NO: 6.

Les inventeurs décrivent également un procédé d'obtention d'un anticorps se liant au domaine extracellulaire de RAMP3, caractérisé en ce qu'il comprend une étape d'immunisation d'un animal avec un peptide choisi parmi les peptides de séquence SEQ ID NO : 5 et SEQ ID NO : 6.

La présente invention a aussi pour objet un procédé d'obtention d'un mélange de trois anticorps se liant aux peptides de séquences SEQ ID NO : 2, 3 et 5, tels que définis ci-dessus, comprenant :
- une étape d'immunisation d'un animal non-humain avec le peptide de séquence SEQ ID NO : 2,
- une étape d'immunisation d'un animal non-humain avec le peptide de séquence SEQ ID NO : 3, et
- une étape d'immunisation d'un animal non-humain avec le peptide de séquence SEQ ID NO : 5.

D'autres aspects et avantages de la présente invention apparaîtront à la lecture des exemples qui suivent, qui doivent être considérés comme illustratifs et non limitatifs, ainsi que des figures annexées :

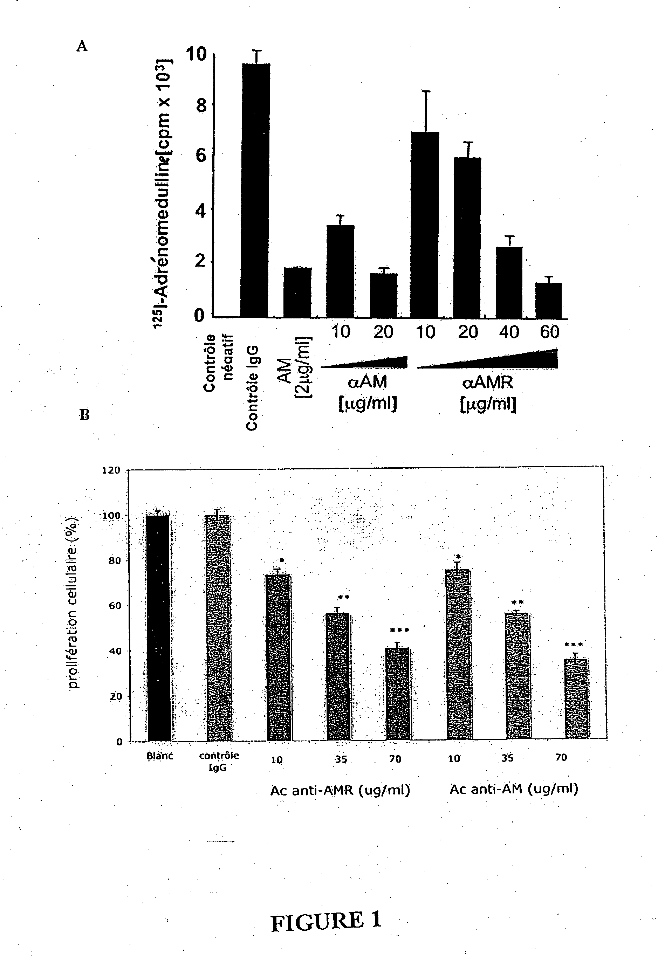

- Figure 1 : Etude de la prolifération cellulaire *in vitro.* A. Effet des anticorps anti-récepteurs de l'adrénomédulline (mélange d'anticorps anti-CRLR, anti-RAMP2 et anti-RAMP3) et anti-adrénomédulline sur la liaison de l'adrénomédulline marquée à l'iode 125 radioactif sur les membranes des cellules gliales U87. Avec 60 µg d'anticorps anti-récepteurs de l'adrénomédulline, l'inhibition de liaison de l'I¹²⁵-AM est plus importante qu'avec 10 µg d'anticorps. B. Dans la lignée issue de glioblastome (U87), le traitement pendant 6 jours avec l'anticorps anti-AM ou le mélange des anticorps anti-CRLR, anti-RAMP2 et anti-RAMP3, inhibe la prolifération cellulaire de 30 à 60% d'une façon dose dépendante, par rapport aux cellules contrôles incubées en présence du sérum pré-immun de lapin. *Test ANOVA:* **, *p<0,01;* ***, *p<0,001.*

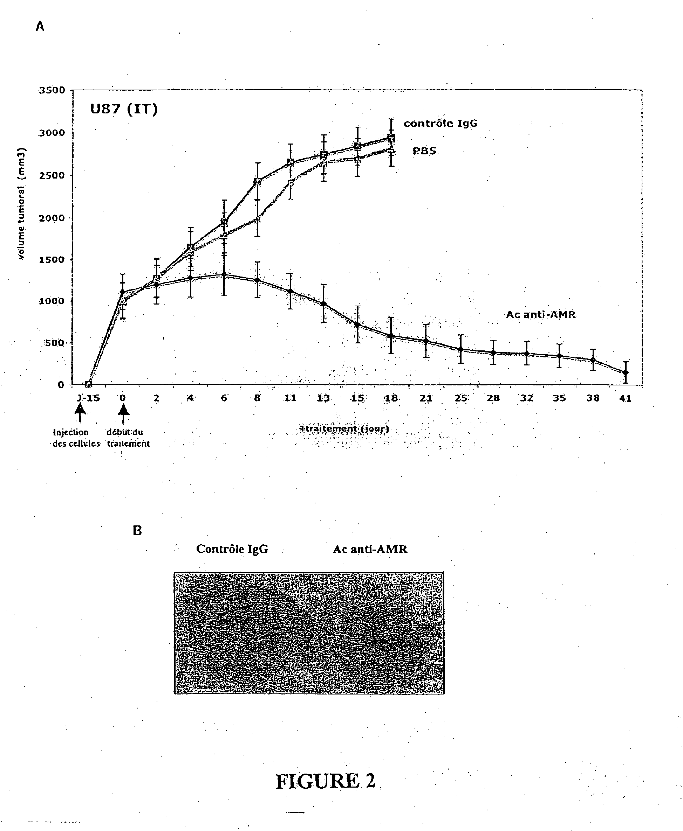

- Figure 2 : L'administration intratumorale des anticorps anti-AMR inhibe la croissance tumorale *in vivo.* A. L'administration intra-tumorale du mélange des anticorps anti-CRLR, anti-RAMP2 et anti-RAMP3 (250 µg/animal) induit une inhibition de la croissance tumorale des xénogreffes de 60-70% après 21 jours de traitement par rapport aux souris contrôles traitées avec le sérum pré-immun du lapin (n=10). *Test ANOVA:* **, *p<0,01; ***, p<0,001.* B. Photos des tumeurs contrôles et traitées à 16 jours après traitement.

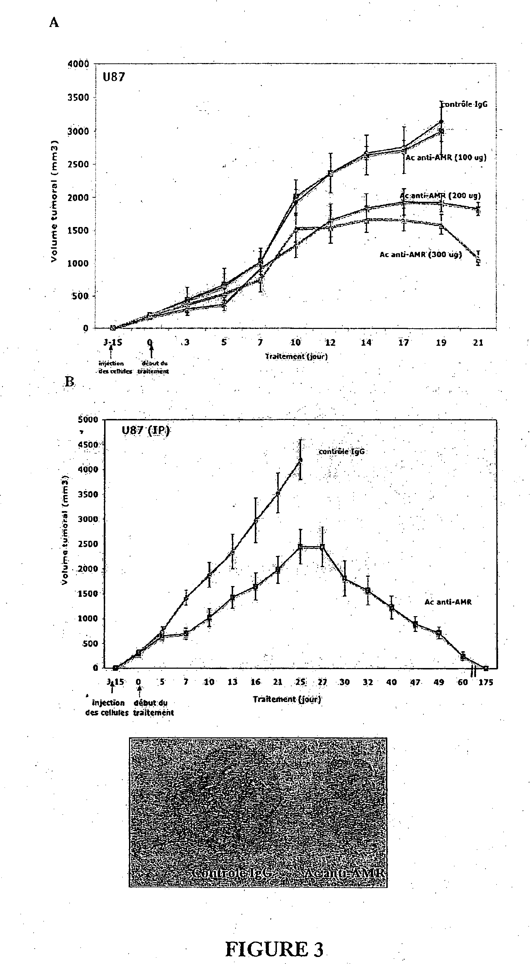

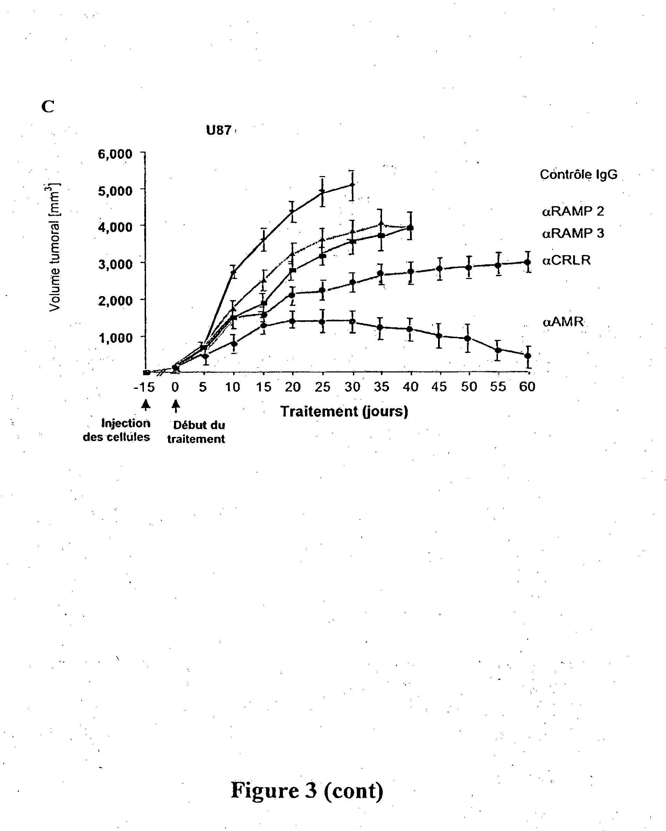

- Figure 3 : L'administration intrapéritonéale des anticorps anti-AMR inhibe la croissance *tumorale in vivo.* A. Dans le but d'établir un traitement par voie intrapéritonéale, différentes doses (100, 200 et 300 µg) du mélange des anticorps anti-AMR ont été testé chez des souris xénogréffées avec la lignée U87 (n=8). B. L'injection par voie intrapéritonéale de 330 µg/souris d'un mélange d'anticorps anti-CRLR, anti-RAMP2 et anti-RAMP3 inhibe la croissance tumorale et augmente la survie de la souris jusqu'à 175 jours par rapport aux souris traitées avec les IgG contrôles qui sont mortes entre 20-25 jours après traitement (n=10). C. Des souris athymiques (nu/nu) xénogréffées avec la lignée U87 ont été traitées avec les anticorps anti-CRLR, anti-RAMP2, anti-RAMP3, le mélange d'anticorps anti-CRLR/anti-RAMP2/anti-RAMP3 ou les IgG contrôles de lapin. Le volume tumoral a ensuite été mesuré. *Test ANOVA:* **, *p<0,01;* ***, *p<0,001.*

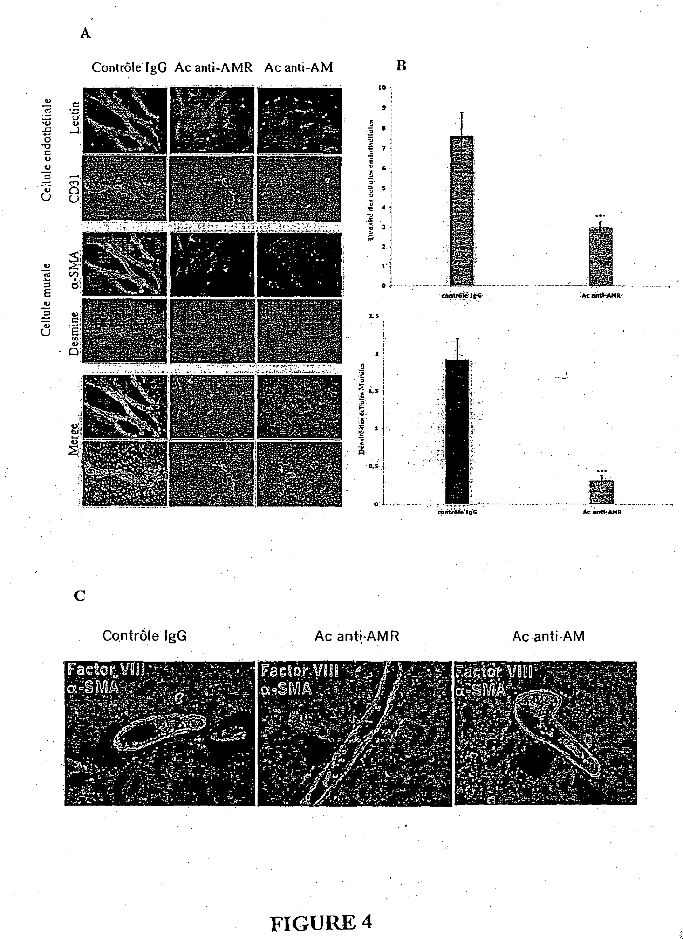

- Figure 4 : Les anticorps anti-AMR déstabilisent la vascularisation tumorale *in vivo.* **A.** Les analyses histologiques réalisées sur des coupes de tumeurs des souris ayant reçu par injection de la lectine biotinylée (marqueur ayant une haute affinité pour la cellule endothéliale) et après révélation à la streptavidine montrent une déstabilisation de l'architecture vasculaire se traduisant par une diminution de la taille des vaisseaux par rapport aux tumeurs contrôles. L'étude immunohistochimique avec des marqueurs des péricytes (Desmine ou α-SMA) montre une diminution très significative voire une disparition des péricytes au niveau des vaisseaux des tumeurs traitées par rapport aux tumeurs contrôles. **B.** La quantification de la vascularisation et la densité cellulaire (cellules endothéliales versus péricytes) entre les 2 groupes d'animaux montre une diminution importante du nombre des cellules endothéliales et des péricytes marquées par unité de surface chez les tumeurs traitées avec les anticorps anti-AMR. *Test ANOVA:* **, *p< 0,01;* ***, *p< 0,001.* C. Le marquage par immunohistochimie des cellules endothéliales (FvIII) /péricytes (α-SMA) montre l'absence des effets du traitement avec les anticorps anti-AMR par voie générale, sur l'architecture vasculaire « normale » au niveau du rein de la souris.

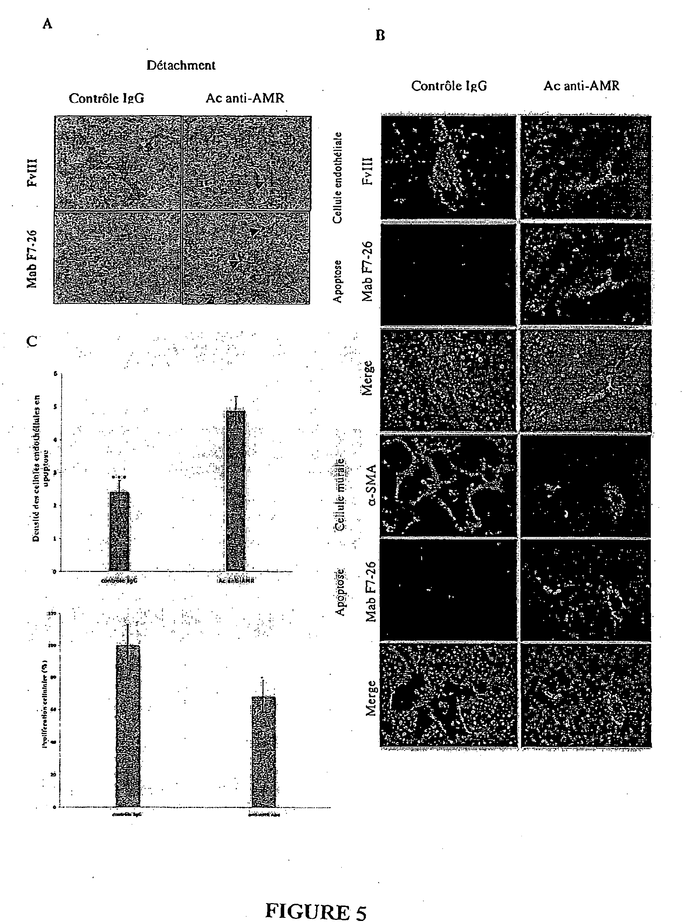

- Figure 5 : Les anticorps anti-AMR induisent l'apoptose des cellules endothéliales et des péricytes. **A.** Le marquage à l'aide de l'anticorps Mab F7-26 montre que les cellules endothéliales détachées de l'endothélium, sous l'effet de l'absence des péricytes, causé par le traitement avec les anticorps anti-AMR, subissent de l'apoptose. **B.** Le marquage des cellules endothéliales (FvIII) et des péricytes (α-SMA) en parallèle avec le marquage à l'aide de l'anticorps Mab F7-26 montre que les péricytes, comme les cellules endothéliales sont en état d'apoptose dans les tumeurs traitées avec les anticorps anti-AMR par rapport aux tumeurs contrôles. C. La quantification de la densité des cellules endothéliales en apoptose montre une augmentation significative dans les tumeurs traitées avec les anticorps anti-AMR par rapport aux tumeurs contrôles. Le marquage de la prolifération cellulaire générale au 16^{ème} jour de traitement ne montre qu'une diminution de 25-35% dans les tumeurs traitées par rapport aux tumeurs contrôles. *=P< 0,05, **=P < 0,01, ***=p< 0,001.

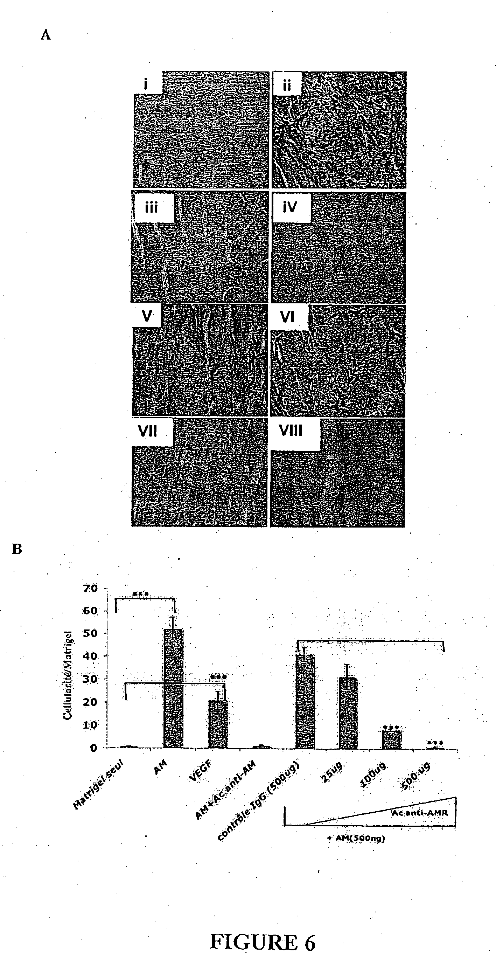

- Figure 6 : L'adrénomédulline active la migration cellulaire d'une façon autocrine/paracrine lors du processus angiogénique *in vivo.* **A.** Le marquage immunohistochimique à l'hématoxyline/éosine sur des coupes d'implants du Matrigel inclus en paraffine montre un envahissement cellulaire important au sein du Matrigel contenant l'adrénomédulline par rapport à celui qui ne contient aucun facteur. Le traitement par de mélange d'anticorps anti-AMR induit une diminution du recrutement des cellules circulantes dans les implants de Matrigel et ce de façon dose dépendante (n=10 par groupe). (I) : Matrigel seul, (II): Matrigel+AM (500 ng), (III): Matrigel + VEGF (500 ng), (IV): Matrigel+AM (500ng)+Ac anti-AM (500 µg), (V): traitement avec les IgG contrôles (500 µg), (VI), (VII) et (VIII) : traitement avec les anticorps anti-AMR (25 µg, 100 et 500 (g). **B**. Quantification du nombre des cellules par unité de surface des coupes du Matrigel. *=P< 0,05, **=P < 0,01, ***= p< 0,001. (N=10).

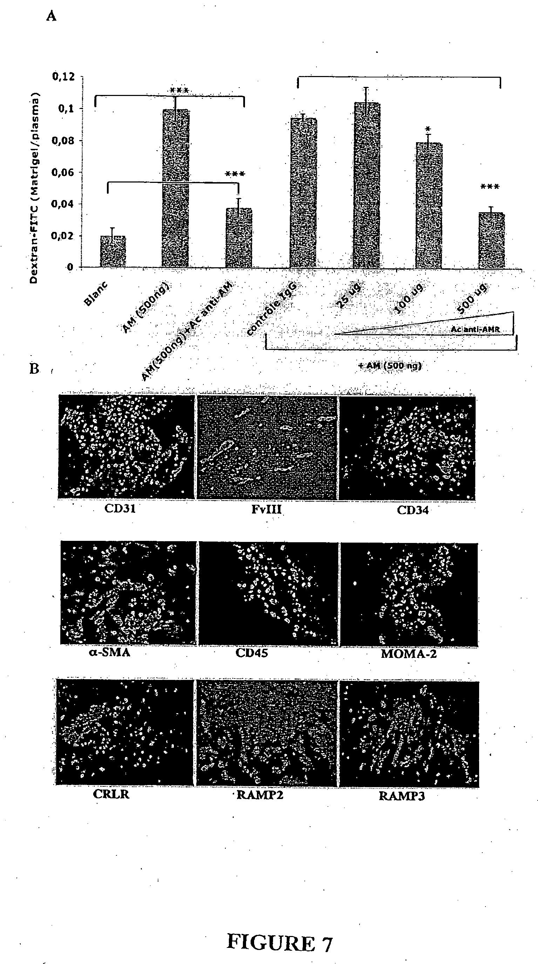

- Figure 7 : Effet de l'adrénomédulline sur le processus de l'angiogenèse *in vivo.* **A.** Le dosage de Dextran-FITC injecté chez la souris ayant des implants de Matrigels en sous-cutané montre une quantité importante de Dextran dans le Matrigel contenant l'adrénomédulline par rapport au contrôle. La quantité du FITC-Dextran diminue d'une façon dose-dépendante chez les souris traitées avec les anticorps anti-AMR par rapport aux souris traitées avec les IgG contrôles. Test ANOVA: **, p< 0,01; ***, p< 0,001. **B.** Le marquage à l'aide des anticorps anti-CD31, anti-FvIII, anti-CD34 (cellules endothéliales et leurs précurseurs), anti-αSMA (péricytes), anti-CD45 et MOMA-2 (leucocytes, monocytes/macrophages) montre la présence de ces différents types cellulaires dans les implants de Matrigel. Le marquage avec les anticorps anti-CRLR, anti-RAMP2 et anti-RAMP3 montre une co-expression des récepteurs de l'adrénomédulline avec les différents marquages utilisés ci-dessus.

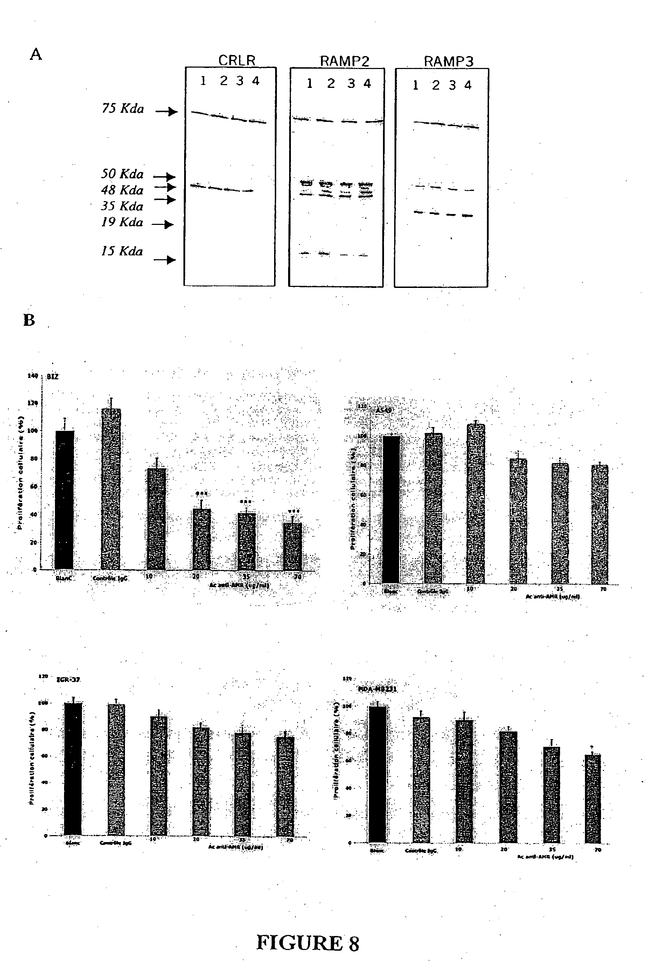

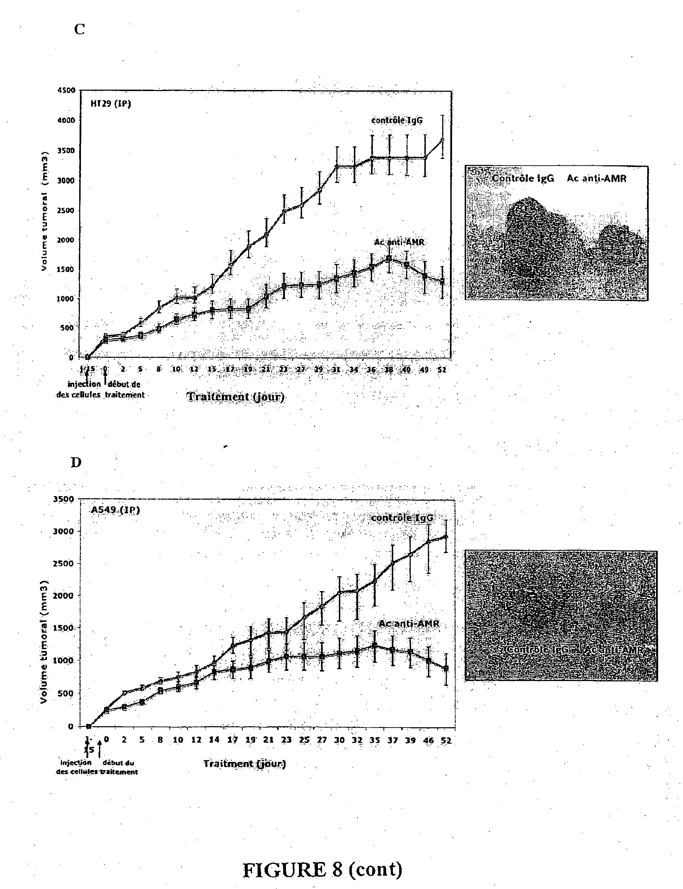

- Figures 8 : Adrénomédulline comme cible thérapeutique. L'analyse par western blot sur des extraits protéiques des lignées tumorales, A549 (1), MDA231 (2), IGR37 (3) et BiZ (4) montre les différentes formes protéiques des récepteurs de l'adrénomédulline **(A).** Les complexes CRLR/RAMP2 et CRLR/RAMP3 (≈ 75 Kda), CRLR (48 Kda), RAMP2 (35 Kda = forme glycosylée, 15 Kda = forme native) et RAMP3 (19 Kda = forme native). Il a aussi été détecté les formes homodimères RAMP2/RAMP2 et RAMP3/RAMP3 (≈ 50 Kda). Le test de prolifération *in vitro* montre que le traitement avec les anticorps anti-AMR (mélange des trois anticorps anti-CRLR, anti-RAMP2 et anti-RAMP3) inhibe la prolifération cellulaire jusqu'à 75% chez la lignée cellulaire du rein (BIZ) **(B).** Chez les autres lignées, A549, IGR-37, et MDA-MB 231 l'inhibition de la prolifération est de 5-30% **(B).** *Test ANOVA: *P<0,05,* **, *p< 0,01;* ***, *p< 0,001.* Le traitement par voie intrapéritonéale avec les anticorps anti-AMR (330 µg/animal) chez les souris xénogréffées avec les cellules HT29 (C) et A549 **(D)** montre une inhibition importante de la croissance tumorale par rapport aux souris traitées par le sérum pré-immun (N=10). *Test ANOVA: **, p<0,01; ***, p<0,001.*

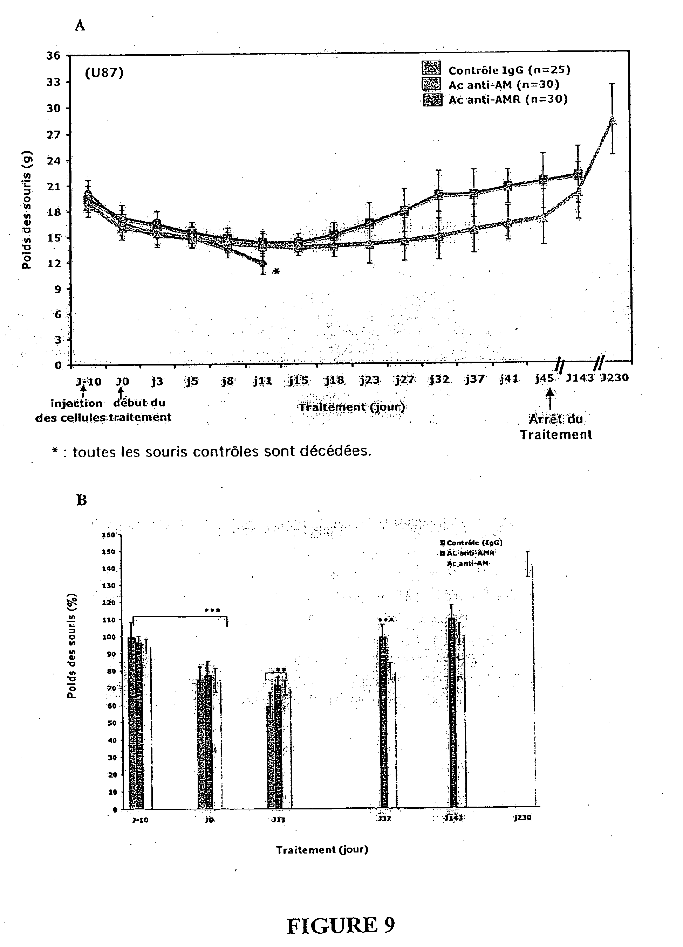

- Figure 9 : Les anticorps anti-AM ou anti-AMR inhibent la croissance tumorale des xénogreffes gliales développées en orthotopie. **A.** Les souris ayant reçus par injection des cellules U87 par voie intracérébrale, montrent une augmentation importante du poids après 5-10 jours du traitement par voie intrapéritoneale avec les anticorps anti-AM ou anti-AMR (330 µg/animal). Ces souris montrent aussi un prolongement de survie d'environ 12 fois plus que les souris traitées par le sérum pré-immun (N=10). **B.** Le poids des souris exprimé en pourcentage montre l'importance des variations du poids après l'injection des cellules tumorales U87 en orthotopie ainsi que pendant le traitement. *Test ANOVA: **, p<0,01; ***, p<0,001.*

### EXEMPLE

### I : MATERIEL ET METHODES

### I.1. Obtention des anticorps anti-récepteurs de l'adrénomédulline.

### Immunisations

Les anticorps polyclonaux anti-CRLR ont été développés par injection chez le.lapin des séquences peptidiques SEQ ID NO : 1 ou SEQ ID NO : 2. Les anticorps polyclonaux anti-RAMP2 ont été développés par injection chez le lapin des séquences peptidiques SEQ ID NO : 3 ou SEQ ID NO : 4. Les anticorps polyclonaux anti-RAMP3 ont été développés par injection chez le lapin des séquences peptidiques SEQ ID NO : 5 ou SEQ ID NO : 6.
SEQ ID NO : 1 : SPEDSIQLGVTRNKIMTAQYEAYQK,
SEQ ID NO : 2 : PDYFQDFDPSEKVTKIADQDGNWFRHPASNR ,
SEQ ID NO : 3 : KNYETAVQFAWNHYKDQMDPIEK,
SEQ ID NO : 4 : RPYSTLRDALEHFAELFDLGFPNPLAER,
SEQ ID NO : 5 : LERLPLAGKAFADMMGKVDVWK,
SEQ ID NO : 6 : GFITGIHRQFFSNATVDRVHLE.

Les animaux ont été immunisés avec les différents peptides complétés avec l'adjuvant de Freund's. Des rappels d'immunisation ont été réalisés par la suite toutes les 3 semaines.

Les sérums non immuns servant de contrôle (pré-immun), ont été prélevées chez les mêmes animaux avant le début des injections.

### Purification des immunoglobulines (IgG) et dosage de l'endotoxine

Les anticorps polyclonaux ont été purifiés par passage sur gel de billes de sépharose couplées à la protéine A (GE Healthcare) et élués à l'aide de glycine 100 mM/pH 3. La présence d'endotoxine dans les anticorps a été vérifiée à l'aide du test LAL (Limulus Amebocyte Lysate, Chambrex). Les résultats montrent un niveau tolérable de l'endotoxine (<1,25 U) dans les différentes préparations d'anticorps ainsi que dans le sérum pré-immun. Le calcul de la concentration en immunoglobuline a été effectué par la méthode Pierce (Bicinchoninic (BCA) Protein Assays ; SMITH et al., Anal Biochem, 1985, 150:76-85).

### 1.2. Culture cellulaire

Les lignées A498 et BIZ proviennent respectivement de DSMZ (Allemagne) et du laboratoire du Dr. Gogusev (Hôpital Necker-Paris). La lignée BIZ dérive d'un cancer du rein ; elle présente une délétion de la région 3p13 pter ainsi que d'autres altérations génétiques telles que der(1) dup(1)(q21 qter) x 2, der(1) t(1;15) x 2, der(13) t(1;13) x 2. Toutes les autres lignées cellulaires proviennent de l'American Type Culture Collection (Rokville MD, USA). Les cellules provenant de tumeurs ou biopsies sont maintenues dans un milieu approprié selon le type cellulaire (cf Tableau 1 ci-dessous), en atmosphère humide composée de 5% CO₂ et 95% air à 37°C.

**Tableau 1 : Cellules utilisées provenant de tumeurs ou biopsies**

| Nom | Provenance | Localisation | Essai chez la souris | Milieu de culture |
|---|---|---|---|---|
| A498 (tumeur) | No ATCC : HTB-44 | Rein | | MEM |
| | | | + | 2 mM glutamine |
| | DSMZ (Allemagne) | | | 1.5 g/L sodium bicarbonate |
| | Homme (52 ans) | | | 1 mM sodium pyruvate 10% FBS |
| A549 (tumeur) | No ATCC : CCL-185 | Poumon | + | DMEM |
| | | | | 2 mM glutamine |
| | homme (58 ans) | | | 10% FBS |
| BIZ (métastase) | Dr. Gogusev INSERM U507-Hôpital Necker-Paris | Rein | Non Testé | RPMI |
| | | | | 2 mM glutamine |
| | | | | 2,2 g/L sodium bicarbonate |
| | | | | 10% FBS |
| Caki1 (métastase) | No ATCC : HTB-46 | Rein | | McCoy's5a |
| | | | + | 2 mM glutamine |
| | homme (49 ans) | | | 2,2 g/L sodium bicarbonate |
| | | | | 10% FBS |
| Caki2 (tumeur) | No ATCC : HTB-47 | Rein | | McCoy's5a |
| | | | + | 2 mM glutamine |
| | homme (69 ans) | | | 2,2 g/L sodium bicarbonate |
| | | | | 10% FBS |
| IGR-37 (tumeur) | No DSMZ : ACC 237 | peau | | MEM |
| | | | + | 2 mM glutamine |
| | homme (59 ans) | | | 10% FBS |
| HT29 (tumeur) | No ATCC: HTB-38 | Colon | | DMEM |
| | | | + | 2 mM glutamine |
| | Femme (44 ans) | | | 10% FBS |
| MCF-7 (métastase) Hormono-sensible | No ATCC : HTB-22 | Sein | | DMEM/F-12 |
| | | | + | 2 mM glutamine insuline 16ng/ml |
| | Femme (69 ans) | | | |
| | | | | 10% FBS |
| MDA-MB231 (métastase) | No ATCC : HTB-26 | Sein | | L-15 |
| | | | + | Insuline 5 µg/ml |
| Hormono-independant | Femme (51 ans) | | | 2 mM glutamine |
| | | | | 10% FBS |
| U87 (tumeur) | No ATCC : HTB-14 | Glioblastome | | MEM |
| | | | + | 2 mM glutamine |
| | homme (68 ans) | | | 1 mM sodium pyruvate |
| | | | | 10% FBS |

Les milieux sont renouvelés tous les deux jours ; lorsque les cellules atteignent 90% de confluence, elles sont détachées avec une solution de Trypsine (0,25%) en tampon Tris (Gibco) pendant quelques minutes à 37°C. L'action de l'enzyme est arrêtée par addition de milieu contenant du sérum. Les cellules sont ensemencées soit dans des tubes de 75 cm² soit dans des plaques multipuits dans leurs milieux appropriés.

### I.3. Spécificité de liaison de l'adrénomédulline à ses propres récepteurs par des expériences de liaison.

Les cellules tumorales gliales U87 sont ensemencées dans des plaques de 24 puits (40000 cellules/puits) et maintenues dans du MEM en présence de 10% de sérum foetal de boeuf (FBS) pendant 48 heures. Elles sont lavées dans du PBS IX et pré-incubées 30 min avec du MEM 0,1% BSA (sérum albumine bovine) contenant l'adrénomédulline radioiodée (Amercham, Biosciences GE) à raison de 100000 cpm en présence d'un mélange d'anticorps composé des anticorps anti-CRLR, anti-RAMP2, et anti-RAMP3. Les anticorps anti-CRLR, anti-RAMP2 et anti-RAMP3 ont été développés chez le lapin par injection respectivement des séquences peptidiques SEQ ID NO: 2, 3 et 5. Après 1 heure d'incubation à température ambiante, les cellules sont placées sur la glace et rincées deux fois avec une solution PBS 1X-0,1%BSA maintenue à 4°C. Les cellules sont solubilisées par de la soude 0,2 N. L'¹²⁵I-AM liée est comptée dans un compteur gamma Riastar (Packard Instrument Compagny).

### 1.4. Etudes in Vivo.

### Modèles animaux

Des souris femelles Balb/C athymique (nu/nu) et des souris C57BL/6 (Harlan, France) âgées de 4-5 semaines ont été utilisées. Elles sont maintenues dans des conditions stériles, température stable et alimentations adaptées. Les expériences *in vivo* ne commencent qu'après une période d'adaptation des animaux à leur nouveau climat (10-15 jours après la réception).

### Développement des xénogreffes et traitements des animaux

Les différentes lignées tumorales U87, A549, HT29 ont été injectées en sous-cutané dans le flanc de souris athymiques (nu/nu) à raison de 2,5x10⁶ cellules par animal. Les animaux sont pesés régulièrement et le volume tumoral est mesuré 3 fois par semaine et calculé selon la formule de l'ellipsoïde V= Longueur x largueur x épaisseur x 0,5236 mm³.

Lorsque les tumeurs atteignent un volume tumoral de 500-1000 mm³ (12-15 jours après injection des cellules), les animaux sont traités par voie intra-tumorale ou intrapéritonéale avec les anticorps anti-CRLR, anti-RAMP2, anti-RAMP3 ou un mélange composé des anticorps anti-CRLR, anti-RAMP2 et anti-RAMP3 dont la concentration finale est de 330 µg/animal et ce à raison de 3 injections/semaine. Les anticorps anti-CRLR, anti-RAMP2 et anti-RAMP3 ont été développés chez le lapin par injection respectivement des séquences peptidiques SEQ ID NO : 2, 3 et 5. Les groupes d'animaux servant de contrôle sont traités de la même manière avec un anticorps non relevant ou un sérum pré-immun.

Les animaux sont sacrifiés à des temps variables au cours du traitement (j2, j7, j 11, j 16, et j21), les tumeurs sont immédiatement prélevées et fixées dans le formol. Elles sont ensuite incluses dans de la paraffine pour les études immunohistochimiques.

Un groupe d'animaux traités pendant 21 jours, reçoit une injection de lectine biotinylée (Biotinylated-lycopersicon Esculentum (tomato) lectin, CliniSciences) sous anesthésie. Les animaux sont perfusés avec une solution de paraformaldéhyde 4% qui permet la fixation des tissus *in vivo.* Les tumeurs et plusieurs organes (cerveau, poumon, coeur et rein) sont prélevés et congelés dans l'Azote liquide pour des études d'histologie et d'immunohistochimie.

### Angiogenèse in vivo

Trois groupes de souris C57BL/6 sont implantés en sous-cutané avec une solution de Matrigel dépourvu en facteurs de croissance (BD Biosciences).
* Groupe (1) Matrigel seul.
* Groupe (2) Matrigel contenant 500 ng de VEGF₁₆₅ (R&D systems, France).
* Groupe (3) Matrigel contenant 500 ng d'adrénomédulline (Bachem).

Après 48 heures, les animaux du groupe (3) sont séparés en 3 sous-groupes qui sont traités 3 fois par semaine par voie intrapéritonéale avec un mélange des anticorps anti-CRLR, anti-RAMP2 et anti-RAMP3. On distingue, le sous-groupe (1) : les animaux traités (25 µg/animal), le sous groupe (2) : les animaux traités (100 µg/animal) et le sous groupe (3) : Animaux traités (500 µg/animal).

En parallèle, un 4^{ème} sous-groupe d'animaux est traité avec le sérum pré-immun (IgG) à la dose de 500 µg/animal.

Après 21 jours du traitement, les animaux sont randomisés et séparés en 2 sous-groupes. Dans le premier sous-groupe, les animaux sont sacrifiés et les implants de Matrigel sont récupérés et fixés dans le formol, puis inclus en paraffine pour les analyses histologiques. Les animaux du deuxième sous-groupe sont injectés sous anesthésie avec du Dextran-FITC (Sigma, France) et sacrifiés après 30 minutes. Les implants de Matrigel sont ensuite traités par la Dispase (Roche), centrifugés à 5000 rpm à 4°C. Les surnageants sont récupérés et la fluorescence est lue à 492 (excitation)-512 nm (émission).

### I.5. Analyses par Western blot.

### Préparation des extraits protéiques

Les culots cellulaires provenant de cellules tumorales gliales U87, les homogénats obtenus à partir de tumeurs gliales xénogreffées chez les souris *nudes* ou de tumeurs de patients atteints de glioblastomes sont repris dans un tampon de lyse (20mM HEPES pH 7,9, 10 mM NaCl, 1 mM MgCl₂, 10% glycérol, 0,2 mM EDTA, 0,5 mM DTT, 1% inhibiteurs des protéases et 0,35% de TritonX-100) et homogénéisés à 4°C. Après centrifugation à 12 000xg pendant 10 minutes, le surnageant contenant les protéines est récupéré et les protéines quantifiées par la méthode de Pierce.

### Western blot

Les lysats cellulaires (50 µg) sont séparés par électrophorèse sur gel de polyacrylamide 12% en conditions dénaturantes et réductrices. A l'issue de la migration dans un tampon Tris-base 0,25 M, Glycine 1,92 M, SDS 1%. Les protéines sont transférées sur membrane PVDF à 1mA/cm2 pendant 1h30. Les membranes sont saturées 1h à température ambiante dans du PBS-5% de lait écrémé. Après 2 lavages (PBS 0,2%-Tween 20), les membranes sont incubées sous agitation pendant une nuit à 4°C en présence des anticorps anti-CRLR, anti-RAMP2 ou anti-RAMP3 dilués à 1/400 dans du PBS-1% lait écrémé. Après 3 lavages (PBS-0,2%-Tween 20), les membranes sont incubées pendant 1h30 à température ambiante avec l'anticorps secondaire marqué à la peroxydase (ECL kit, GE Healthcare, Amersham). Le signal est révélé par le kit de chimioluminescence (ECL kit, GE Healthcare, Amersham).

### I.6. Etudes immunohistochimiques.

Les différentes analyses histologiques ont été faites sur des coupes de 6 µm de tumeurs congelées (cryostat) ou après inclusion en paraffine (microtome). Les coupes sont de 30-50 µm pour les tumeurs des souris injectées par la lectine biotinylée.

Les coupes sont déparaffinées après un bain de xylène suivi par 3 bains d'éthanol (100%, 95% et 75%). Après un lavage au PBS, les sites non spécifiques sont saturés par le sérum du kit Vectastain (Abcys). Les coupes sont ensuite incubées pendant une nuit avec l'anticorps primaire. Les différents anticorps utilisés sont : l'anti-facteur VIII (Dako, 1:300), l'anti-CD31 (Dako, 1 : 40), l'anti-CD34 (Zymed laboratories), l'anti-αSMA (Dako, 1 :100), l'anti-NG2 (Chemicon, 1 :150) et l'anti-desmine (Abcam, 1 :50). Les leucocytes et les monocytes/macrophages ont été détectés à l'aide des anticorps anti-CD45 (BD Pharmingen, 1: 40) et l'anticorps MOMA-2 (Chemicon, 1: 25).

Pour le marquage des cellules en apoptose, l'anticorps Mab F7-26 (AbCys) a été utilisé et pour la prolifération cellulaire, l'anticorps anti-Ki67 (Dako, 1 : 80).

Après 3 lavages avec le tampon de phosphate 0,1 M, pH 7,4, les coupes sont incubées à température ambiante pendant 1h 30 avec le Dapi (Invitrogen, 1/30000) et les anticorps secondaires couplés à des fluorochromes (Invitrogen : 1/250). La lectine biotinylée est révélée par l'anticorps secondaire streptavidine-Alexa fluor (Invitrogen, 1/250). Après 3 lavages, les coupes sont couvertes par des lamelles. Les acquisitions des photos sont effectuées à l'aide d'un microscope Zeiss et le logiciel Leika.

### I.7. Analyses Statistiques.

Toutes les expériences ont été répétées 3 à 4 fois. L'analyse statistique a été réalisée par le test Anova/test-S. Les résultats sont considérés comme significatifs à partir de P < 0,05.

### II : RÉSULTATS

### II.1 Les anticorps anti-AMR (mélange d'anticorps anti-CRLR, anti-RAMP2 et anti-RAMP3) inhibent la prolifération des cellules gliales in vitro

Lors de la caractérisation des anticorps anti-récepteurs de l'adrénomédulline, il a été démontré que ces anticorps sont capables d'inhiber d'une façon dose-dépendante la liaison de l'adrénomédulline radioactif « ¹²⁵I-AM » sur des préparations membranaires provenant de cellules tumorales (Figure 1 A).

Ces *expériences in vitro* permettent aussi de mettre en évidence la présence d'une boucle autocrine et/ou paracrine faisant intervenir l'adrénomédulline et ses récepteurs CRLR, RAMP2 et RAMP3 dans la prolifération des cellules tumorales (Figure 1 B). Ces données démontrent aussi que le mélange d'anticorps anti-CRLR, anti-RAMP2 et anti-RAMP3 reconnaissent le récepteur de l'adrénomédulline et bloquent, par conséquent, la prolifération de ces cellules due à l'action de l'adrénomédulline sécrétée par ces mêmes cellules.

### II.2. Les anticorps anti-AMR (mélange d'anticorps anti-CRLR, anti-RAMP2 et anti-RAMP3) inhibent la croissance tumorale gliale in vivo.

Les tumeurs développées chez les souris athymiques après injection en sous-cutané de lignées cellulaires (U87) représentent un modèle expérimental qui prend en compte toutes les composantes du micro-environnement tumoral.

### Administration des anticorps en intra-tumorale

L'administration intra-tumorale du mélange d'anticorps anti-CRLR, anti-RAMP2 et anti-RAMP3 induit une inhibition de la croissance tumorale des xénogreffes de 60-70% après 21 jours de traitement (Figure 2 A). Après 16 jours de traitement, il a été observé que les tumeurs d'animaux traités avec les anticorps anti-AMR apparaissaient pâles, translucides et moins vascularisées (Figure 2 B). A l'opposé, les animaux contrôles présentent des tumeurs larges très vascularisées. Ces effets importants observés *in vivo* laissent penser que, outre une action sur la prolifération des cellules tumorales, le traitement avec les anticorps anti-AMR perturbe un mécanisme fondamental indispensable à la croissance tumorale.

### Administration des anticorps par voie intrapéritonéale

Afin évaluer l'effet thérapeutique des anticorps anti-AMR sur la croissance tumorale *in vivo,* les anticorps anti-AMR ont été administrés par voie intrapéritonéale.

Quatre groupés de 8 souris chacun ont été traitées 3 fois par semaine avec des IgG contrôles (330 µg), anti-AMR (100, 200, 330 µg) (Figure 3 A).

Ces résultats montrent une inhibition dose-dépendante de la croissance tumorale gliale après traitement avec les anticorps anti-AMR. Pour la suite des études, il a été adopté la concentration de 330 µg avec le même protocole d'injection.

Le traitement par voie intrapéritonéale montre une inhibition très importante de la croissance tumorale gliale et une survie beaucoup plus importante des souris traitées avec les anticorps anti-AMR par rapport aux souris traitées aux IgG contrôles (Figure 3 B).

Le traitement par voie intrapéritonéale montre aussi une inhibition très importante de la croissance tumorale gliale et une survie beaucoup plus importante des souris traitées avec le mélange des trois anticorps anti-CRLR, anti-RAMP2 et anti-RAMP3 par rapport aux souris traitées avec un seul anticorps anti-CRLR, anti-RAMP2 ou anti-RAMP3 (Figure 3 C).

### II.3. Les anticorps anti-AMR (mélange d'anticorps anti-CRLR, anti-RAMP2 et anti-RAMP3) déstabilisent la vascularisation tumorale in vivo.

Afin de mieux comprendre les mécanismes impliqués dans la régression tumorale après traitement avec les anticorps anti-AMR (anti-CRLR/anti-RAMP2/anti-RAMP3), des analyses histologiques ont été réalisées sur des coupes de tumeurs. L'utilisation des marqueurs endothéliaux comme le CD31 ou le facteur de von Willebrand (vWF) atteste d'une désorganisation profonde voire une déstabilisation de l'architecture vasculaire se traduisant par une diminution de la taille des vaisseaux.

L'injection de la lectine biotinylée (marqueur ayant une haute affinité pour la cellule endothéliale) chez la souris 15 minutes avant le sacrifice, montre une vascularisation stable et fonctionnelle chez les animaux traités aux IgG contrôle, alors qu'une désorganisation vasculaire a été observée chez les animaux traités avec les anticorps anti-AMR. D'une façon intéressante, l'étude immunohistochimique avec des marqueurs des péricytes (NG2, Desmine ou α-SMA) montre une diminution très significative voire une disparition des péricytes au niveau des vaisseaux des tumeurs traitées par rapport aux tumeurs contrôles (Figure 4 A). La quantification de la vascularisation et la densité cellulaire (cellules endothéliales versus péricytes) entre les 2 groupes d'animaux montre une diminution significative dans les tumeurs traitées (Figure 4 B). Les expériences suggèrent ainsi que l'adrénomédulline régule la couverture des vaisseaux par les péricytes et que les anticorps anti-AMR sont capables de bloquer le recrutement des péricytes au niveau des vaisseaux. Ceci révèle que les anticorps anti-AMR induisent une régression de la vascularisation tumorale probablement par la déstabilisation des vaisseaux après la perte des cellules de soutien, les péricytes.

L'analyse par immunohistochimie de la vascularisation de différents organes (rein, coeur, poumon, *etc...*) à l'aide d'un co-marquage des cellules endothéliales/péricytes chez les différents groupes des souris traitées avec les anticorps anti-AMR et les souris traitées avec les IgG contrôles montre l'absence des effets du traitement sur l'architecture vasculaire non tumorale (Figure 4 C). Ces résultats révèlent que les anticorps anti-AMR agissent seulement au niveau tumoral sans pour autant avoir des effets sur la vascularisation des différents organes de la souris.

Les péricytes contribuent au processus angiogénique par un effet sur la synthèse ou la dégradation de la matrice extracellulaire, à la stabilité du mur vasculaire en participant à l'assemblage de la membrane basale, et apparaissent aussi comme un facteur de régulation paracrine supprimant la prolifération et la migration des cellules endothéliales (SATO and RIFKIN, J Cell Biol., 1989, 109:309-15 ; BENJAMIN et al., Development, 1998, 125:1591-8). L'architecture de l'arbre vasculaire est contrôlée et stabilisée par le flux sanguin, mais aussi par les interactions qui s'établissent entre les cellules endothéliales, les péricytes, les cellules musculaires lisses et la matrice extracellulaire (ALLT and LAWRENSON, Cells Tissues Organs, 2001, 169:1-11). L'utilisation d'anticorps Mab F7-26 mettant en évidence la présence d'apoptose, montre que les cellules endothéliales détachées de l'endothélium, du fait de l'absence des péricytes, causé par le traitement avec les anticorps anti-AMR, subissent une apoptose (Figure 5 A). Ce même marquage a été observé pour les rares cellules péricytaires présentes dans les tumeurs traitées (Figure 5 B). En revanche, le marquage de la prolifération cellulaire générale au 16^{ème} jour de traitement ne montre qu'une diminution de 25-35% chez les tumeurs traitées par rapport aux tumeurs contrôles (Figure 5 C). Ces résultats mettent l'accent sur le rôle potentiel de l'adrénomédulline sur le recrutement des péricytes, impliquant donc ce type cellulaire comme un composant fonctionnel important de la vascularisation tumorale.

### II.4. Effet du traitement avec les anticorps anti-AMR (mélange d'anticorps anti-CRLR, anti-RAMP2 et anti-RAMP3) par voie intrapéritonéale.

Le test *d'angiogenèse in vivo* à l'aide du Matrigel dépourvu des facteurs de croissance et supplémenté seulement avec de l'adrénomédulline, injecté à des souris C57BL/6 en sous-cutané, est un bon modèle pour étudier l'effet de la présence de ce facteur sur la mobilisation des différents types cellulaires. Le marquage à l'hématoxyline/éosine sur des coupes en histologie montre un envahissement cellulaire au sein du Matrigel contenant l'adrénomédulline en comparaison avec le Matrigel sans aucun facteur. Par ailleurs la densité cellulaire est plus importante si on compare avec le Matrigel contenant le VEGF (Figure 6 A). Pour évaluer le rôle des anticorps anti-AMR (anti-CRLR/anti-RAMP2/anti-RAMP3) sur le recrutement des cellules circulantes, l'effet du traitement avec ces anticorps par voie intra-péritonéale a été testé. Les résultats montrent que le traitement par les anticorps anti-AMR induit une diminution du recrutement des cellules circulantes dans les implants de Matrigel et ce de façon dose-dépendante (Figure 6 A et B). La spécificité des anticorps anti-AMR est prouvée en comparaison au traitement à l'aide des IgG pré-immun.

### II.5. L'effet de l'adrénomédulline sur le processus de l'angiogenèse in vivo.

L'injection de Dextran-FITC chez la souris C57BL/6 ayant reçu en sous-cutané le Matrigel contenant ou non l'adrénomédulline, 30 minutes avant le sacrifice de l'animal, permet d'étudier l'effet de l'adrénomédulline sur l'angiogenèse *in vivo.* Le dosage de FITC fluorescent montre une quantité importante de Dextran dans le Matrigel contenant l'adrénomédulline par rapport au contrôle attestant d'une angiogenèse fonctionnelle mise en place sous l'effet de l'adrénomédulline (Figure 7 A). La quantité du FITC-Dextran diminue d'une façon dose-dépendante chez les souris traitées avec les anticorps anti-AMR par rapport aux souris traitées avec les IgG contrôles.

La formation des vaisseaux sanguins est un processus qui met en oeuvre plusieurs types cellulaires : les cellules endothéliales, qui tapissent la paroi des vaisseaux ; les péricytes, qui stabilisent ces parois ; et les cellules circulantes (cellules inflammatoires, précurseurs de cellules endothéliales et cellules mésenchymateuses). Le marquage à l'aide de différents marqueurs des cellules endothéliales et leurs précurseurs (anti-CD31, anti-FvIII, anti-CD34), des péricytes (anti-αSMA, anti-desmine, anti-NG2) et des cellules inflammatoires (anti-CD45 et MOMA-2) a permis d'identifier les différents types cellulaires attirées par la présence de l'adrénomédulline (Figure 7 B). Le marquage avec les anticorps anti-CRLR, anti-RAMP2 et anti-RAMP3 a permis de différencier les populations cellulaires exprimant les récepteurs de l'adrénomédulline et par conséquent, recrutées sous l'effet paracrine de l'adrénomédulline dans le Matrigel. Par contre, les résultats révèlent aussi la présence des cellules qui n'expriment pas ces récepteurs et qui sont probablement recrutées dans le Matrigel sous l'effet d'autres facteurs qui peuvent être libérés par les différentes cellules après leur contact avec l'adrénomédulline.

Ces résultats montrent que l'adrénomédulline est impliquée, par un effet autocrine/paracrine, dans différentes étapes de la néo-angiogenèse intra-tumorale telles que la migration, l'invasion, et la différenciation cellulaire *via* ses récepteurs. Ainsi, le blocage des récepteurs de l'adrénomédulline (AMR) semble être suffisant pour inhiber la croissance tumorale.

### II.6. Effet du traitement avec les anticorps anti-AMR (mélange d'anticorps anti-CRLR, anti-RAMP2 et anti-RAMP3) sur différents modèles tumoraux

Les différents résultats obtenus définissent l'adrénomédulline comme un facteur impliqué dans l'angiogenèse tumorale.

Dans le but d'établir une thérapie anti-cancéreuse, l'effet du traitement avec les anticorps anti-AMR (anti-CRLR/anti-RAMP2/anti-RAMP3) sur d'autres modèles tumoraux, tels que le cancer de poumon, du côlon, du rein, du sein et de la peau a été vérifié.

L'analyse par la technique de western blot réalisée sur les extraits protéiques des différentes lignées tumorales, A549 pour le cancer du poumon, HT29 pour le cancer du côlon, A498, Caki 1,2 et BIZ pour le cancer du rein, MDA-MB-231 pour le cancer du sein et IGR pour le cancer de la peau, montre la présence des différentes protéines constituant les récepteurs de l'adrénomédulline, CRLR, RAMP2 et RAMP3 (Figure 8 A).

L'étude *in vitro* de l'effet des anticorps anti-AMR (anti-CRLR/anti-RAMP2/anti-RAMP3) sur la prolifération de ces différentes lignées montre une inhibition de la prolifération qui atteint 70% à 70 µg/ml après 6 jours de traitement chez la lignée du rein, BIZ (Figure 8 B). En ce qui concerne les autres lignées, A549, IGR-37 et MDA-MB231, l'inhibition de la prolifération cellulaire est d'environ de 30% avec la même concentration d'anticorps (Figure 8 B). Par ailleurs, ces résultats montrent que la prolifération *in vitro* de la lignée BIZ nécessite la présence de l'adrénomédulline en comparaison aux autres lignées étudiées.

L'investigation a été poursuivie *in vivo* chez la souris nude (Balb-c nu/nu). Pour cela, des xénogreffes hétérotopiques ont été développées en sous cutané.

Le traitement par voie intrapéritonéale avec les anticorps anti-AMR chez les souris xénogréffées avec les lignées HT29 (Figure 8 C) et A549 (Figure 8 D), montre une inhibition importante de la croissance tumorale par rapport aux souris traitées avec le sérum pré-immun. Ces premières observations suggèrent une bonne tolérance générale du traitement par les anticorps anti-AMR (courbe pondérale et état général des souris traitées). Les tumeurs des souris traitées sont 3 fois moins volumineuses que les tumeurs contrôles et apparaissent aussi moins vascularisées. Ces résultats mettent en évidence le rôle important que doit jouer l'adrénomédulline dans le développement des tumeurs malignes. Les études préliminaires d'immunohistochimie sur des coupes des xénogreffes du côlon, par marquage avec le facteur VIII et le CD31 pour la cellule endothéliale et l'α-SMA et la desmine pour le péricyte, montrent un effet très important des anticorps anti-AMR sur l'angiogenèse tumorale.

### II.7. Effet des anticorps anti-AMR (anti-CRLR/anti-RAMP2/anti-RAMP3) sur la croissance tumorale gliale des xénogreffes développées en orthotopie.

Trois groupes de 10 souris chacun ont été injectées par voie intracérébrale avec un million de cellules U87. Dix jours plus tard, les souris affaiblies à cause de la maladie, subissent une perte de poids (14 gr ± 2) par rapport aux souris normales sans injection des cellules (20 gr ± 2). Les souris sont séparées en plusieurs groupes, et ont reçu 3 fois par semaine, 330 µg par voie intrapéritonéale des IgG contrôles ou de l'anticorps anti-adrénomédulline (anti-AM) ou des anticorps anti-récepteurs de l'adrénomédulline (anti-AMR) (Figure 9 A).

Chez les souris ayant reçu par injections les IgG contrôles, la survie était de 5-10 jours après traitement avec une chute spectaculaire de leurs poids (Figure 9 B).

En revanche, dans les groupes des souris traitées avec les anticorps anti-AM ou anti-AMR, on a observé chez 60 à 70% entre elles, un gain de poids quelques jours après le traitement avec une survie qui était prolongée au delà de 230 jours (Figure 9 B).

Enfin, chez les souris traitées, aucune métastase n'a été observée au niveau des organes après le sacrifice de l'animal.

## Revendications

1. Mélange d'au moins trois anticorps et/ou fragments desdits anticorps se liant aux protéines CRLR, RAMP2 et RAMP3 formant les récepteurs de l'adrénomédulline, chaque anticorps et/ou fragments d'anticorps se liant à une protéine différente, et chaque anticorps et/ou fragments d'anticorps étant un antagoniste d'un desdits récepteurs de l'adrénomédulline, pour utilisation comme médicament.

2. Mélange pour l'utilisation selon la revendication 1, **caractérisé en ce que** le médicament est destiné au traitement préventif ou curatif des tumeurs.

3. Mélange pour l'utilisation selon la revendication 2, **caractérisé en ce que** lesdites tumeurs sont des tumeurs solides.

4. Mélange pour l'utilisation selon l'une quelconque des revendications 1 à 3, **caractérisé en ce que** lesdits anticorps se lient à un domaine extracellulaire de chacune desdites protéines.

5. Mélange pour l'utilisation selon l'une quelconque des revendications 1 à 4, **caractérisé en ce que** lesdits anticorps sont des anticorps polyclonaux ou monoclonaux.

6. Mélange pour l'utilisation selon l'une quelconque des revendications 1 à 5, **caractérisé en ce que** l'anticorps se liant à un domaine extracellulaire de CRLR est obtenu par immunisation d'un animal non-humain avec un peptide choisi parmi les séquences SEQ ID NO : 1 et SEQ ID NO : 2.

7. Mélange pour l'utilisation selon l'une quelconque des revendications 1 à 6, **caractérisé en ce que** l'anticorps se liant au domaine extracellulaire de RAMP2 est obtenu par immunisation d'un animal non-humain avec un peptide choisi parmi les séquences SEQ ID NO : 3 et SEQ ID NO : 4.

8. Mélange pour l'utilisation selon l'une quelconque des revendications 1 à 7, **caractérisé en ce que** l'anticorps se liant au domaine extracellulaire de RAMP3 est obtenu par immunisation d'un animal non-humain avec un peptide choisi parmi les séquences SEQ ID NO : 5 et SEQ ID NO : 6.

9. Composition pharmaceutique, **caractérisée en ce qu'**elle comprend au moins trois anticorps et/ou fragments d'anticorps se liant aux protéines CRLR, RAMP2 et RAMP3 formant les récepteurs de l'adrénomédulline, chaque anticorps et/ou fragments d'anticorps se liant à une protéine différente, et chaque anticorps et/ou fragments d'anticorps étant un antagoniste d'un desdits récepteurs de l'adrénomédulline, et au moins un véhicule pharmaceutiquement acceptable.

10. Composition pharmaceutique selon la revendication 9, **caractérisée en ce que** lesdits anticorps et/ou fragments d'anticorps sont tels que définis à l'une quelconque des revendications 4 à 8.

11. Anticorps se liant à un domaine extracellulaire de CRLR, **caractérisé en ce qu'**il est susceptible d'être obtenu par immunisation d'un animal non-humain avec le peptide de séquence SEQ ID NO : 1.

12. Anticorps se liant au domaine extracellulaire de RAMP2, **caractérisé en ce qu'**il est susceptible d'être obtenu par immunisation d'un animal non-humain avec le peptide de séquence SEQ ID NO : 4.

13. Anticorps se liant au domaine extracellulaire de RAMP3, **caractérisé en ce qu'**il est susceptible d'être obtenu par immunisation d'un animal non-humain avec le peptide de séquence SEQ ID NO : 6.

14. Procédé d'obtention d'un anticorps se liant à un domaine extracellulaire de CRLR, **caractérisé en ce qu'**il comprend une étape d'immunisation d'un animal non-humain avec le peptide de séquence SEQ ID NO : 1.

15. Procédé d'obtention d'un anticorps se liant au domaine extracellulaire de RAMP2, **caractérisé en ce qu'**il comprend une étape d'immunisation d'un animal non-humain avec le peptide de séquence SEQ ID NO : 4.

16. Procédé d'obtention d'un anticorps se liant au domaine extracellulaire de RAMP3, **caractérisé en ce qu'**il comprend une étape d'immunisation d'un animal non-humain avec le peptide de séquence SEQ ID NO : 6.

17. Procédé d'obtention d'un mélange de trois anticorps se liant aux peptides de séquences SEQ ID NO : 2, 3 et 5, tel que défini à l'une quelconque des revendications 1, 4 et 5, comprenant :
- une étape d'immunisation d'un animal non-humain avec le peptide de séquence SEQ ID NO : 2,
- une étape d'immunisation d'un animal non-humain avec le peptide de séquence SEQ ID NO : 3, et
- une étape d'immunisation d'un animal non-humain avec le peptide de séquence SEQ ID NO : 5.

## Patentansprüche

1. Gemisch aus mindestens drei Antikörpern und/oder Fragementen der Antikörper, die an die Proteine CRLR, RAMP2 und RAMP3 binden, welche Adrenomedullinrezeptoren bilden, wobei jeder Antikörper und/oder jedes Antikörperfragment an ein anderes Protein bindet und jeder Antikörper und/oder jedes Antikörperfragment ein Antagonist eines der Adrenomedullinrezeptoren ist, zur Verwendung als Arzneimittel.

2. Gemisch zur Verwendung nach Anspruch 1, **dadurch gekennzeichnet, dass** das Medikament zur präventiven oder kurativen Behandlung von Tumoren bestimmt ist.

3. Gemisch zur Verwendung nach Anspruch 2, **dadurch gekennzeichnet, dass** die Tumoren solide Tumoren sind.

4. Gemisch zur Verwendung nach einem der Ansprüche 1 bis 3, **dadurch gekennzeichnet, dass** die Antikörper an eine extrazelluläre Domäne jedes der Proteine binden.

5. Gemisch zur Verwendung nach einem der Ansprüche 1 bis 4, **dadurch gekennzeichnet, dass** die Antikörper polyklonale oder monoklonale Antikörper sind.

6. Gemisch zur Verwendung nach einem der Ansprüche 1 bis 5, **dadurch gekennzeichnet, dass** der Antikörper, der an eine extrazelluläre Domäne von CRLR bindet, durch Immunisierung eines nichtmenschlichen Tiers mit einem Peptid, ausgewählt aus den Sequenzen SEQ ID NO : 1 und SEQ ID NO : 2, erhalten wird.

7. Gemisch zur Verwendung nach einem der Ansprüche 1 bis 6, **dadurch gekennzeichnet, dass** der Antikörper, der an die extrazelluläre Domäne von RAMP2 bindet, durch Immunisierung eines nichtmenschlichen Tiers mit einem Peptid, ausgewählt aus den Sequenzen SEQ ID NO : 3 und SEQ ID NO : 4, erhalten wird.

8. Gemisch zur Verwendung nach einem der Ansprüche 1 bis 7, **dadurch gekennzeichnet, dass** der Antikörper, der an die extrazelluläre Domäne von RAMP3 bindet, durch Immunisierung eines nichtmenschlichen Tiers mit einem Peptid, ausgewählt aus den Sequenzen SEQ ID NO : 5 und SEQ ID NO : 6, erhalten wird.

9. Pharmazeutische Zusammensetzung, **dadurch gekennzeichnet, dass** sie umfasst: mindestens drei Antikörper und/oder Antikörperfragmente, die an die Proteine CRLR, RAMP2 und RAMP3 binden, welche Adrenomedullinrezeptoren bilden, wobei jeder Antikörper und/oder jedes Antikörperfragment an ein anderes Protein bindet und jeder Antikörper und/oder jedes Antikörperfragment ein Antagonist eines der Adrenomedullinrezeptoren ist, und mindestens einen pharmazeutisch verträglichen Träger.

10. Pharmazeutische Zusammensetzung nach Anspruch 9, **dadurch gekennzeichnet, dass** die Antikörper und/oder Antikörperfragmente wie in einem der Ansprüche 4 bis 8 definiert sind.

11. An eine extrazelluläre Domäne von CRLR bindender Antikörper, **dadurch gekennzeichnet, dass** er durch Immunisierung eines nichtmenschlichen Tiers mit dem Peptid der Sequenz SEQ ID NO : 1 erhalten werden kann.

12. An die extrazelluläre Domäne von RAMP2 bindender Antikörper, **dadurch gekennzeichnet, dass** er durch Immunisierung eines nichtmenschlichen Tiers mit dem Peptid der Sequenz SEQ ID NO : 4 erhalten werden kann.

13. An die extrazelluläre Domäne von RAMP3 bindender Antikörper, **dadurch gekennzeichnet, dass** er durch Immunisierung eines nichtmenschlichen Tiers mit dem Peptid der Sequenz SEQ ID NO : 6 erhalten werden kann.

14. Verfahren zum Erhalt eines an eine extrazelluläre Domäne von CRLR bindenden Antikörpers, **dadurch gekennzeichnet, dass** es einen Schritt der Immunisierung eines nichtmenschlichen Tiers mit dem Peptid der Sequenz SEQ ID NO : 1 umfasst.

15. Verfahren zum Erhalt eines an die extrazelluläre Domäne von RAMP2 bindenden Antikörpers, **dadurch gekennzeichnet, dass** es einen Schritt der Immunisierung eines nichtmenschlichen Tiers mit dem Peptid der Sequenz SEQ ID NO : 4 umfasst.

16. Verfahren zum Erhalt eines an die extrazelluläre Domäne von RAMP3 bindenden Antikörpers, **dadurch gekennzeichnet, dass** es einen Schritt der Immunisierung eines nichtmenschlichen Tiers mit dem Peptid der Sequenz SEQ ID NO : 6 umfasst.

17. Verfahren zum Erhalt eines Gemischs aus drei Antikörpern, die an die Peptide der Sequenzen SEQ ID NO : 2, 3 und 5 binden, wie in einem der Ansprüche 1, 4 und 5 definiert, umfassend:
- einen Schritt der Immunisierung eines nichtmenschlichen Tiers mit dem Peptid der Sequenz SEQ ID NO : 2,
- einen Schritt der Immunisierung eines nichtmenschlichen Tiers mit dem Peptid der Sequenz SEQ ID NO : 3, und
- einen Schritt der Immunisierung eines nichtmenschlichen Tiers mit dem Peptid der Sequenz SEQ ID NO : 5.

## Claims

1. Mixture of at least three antibodies and/or fragments of said antibodies that bind to the CRLR, RAMP2 and RAMP3 proteins forming the adrenomedullin receptors, each antibody and/or antibody fragment binding to a different protein and each antibody and/or antibody fragment being an antagonist of one of said adrenomedullin receptors, for use as a medicament.

2. Mixture for use according to claim 1, **characterised in that** the medicament is intended for the preventive or curative treatment of tumours.

3. Mixture for use according to claim 2, **characterised in that** said tumours are solid tumours.

4. Mixture for use according to any one of claims 1 to 3, **characterised in that** said antibodies bind to an extracellular domain of each of said proteins.

5. Mixture for use according to any one of claims 1 to 4, **characterised in that** said antibodies are polyclonal or monoclonal antibodies.

6. Mixture for use according to any one of claims 1 to 5, **characterised in that** the antibody that binds to an extracellular domain of CRLR is obtained by immunising a non-human animal with a peptide chosen from sequences SEQ ID NO: 1 and SEQ ID NO: 2.

7. Mixture for use according to any one of claims 1 to 6, **characterised in that** the antibody that binds to the extracellular domain of RAMP2 is obtained by immunising a non-human animal with a peptide chosen from sequences SEQ ID NO: 3 and SEQ ID NO: 4.

8. Mixture for use according to any one of claims 1 to 7, **characterised in that** the antibody that binds to the extracellular domain of RAMP3 is obtained by immunising a non-human animal with a peptide chosen from sequences SEQ ID NO: 5 and SEQ ID NO: 6.

9. Pharmaceutical composition, **characterised in that** it comprises at least three antibodies and/or fragments of antibodies that bind to the CRLR, RAMP2 and RAMP3 proteins forming the adrenomedullin receptors, each antibody and/or antibody fragment binding to a different protein and each antibody and/or antibody fragment being an antagonist of one of said adrenomedullin receptors, and at least one pharmaceutically acceptable carrier.

10. Pharmaceutical composition according to claim 9, **characterised in that** said antibodies and/or antibody fragments are as defined in any one of claims 4 to 8.

11. Antibody that binds to an extracellular domain of CRLR, **characterised in that** it is obtainable by immunising a non-human animal with the peptide of sequence SEQ ID NO: 1.

12. Antibody that binds to the extracellular domain of RAMP2, **characterised in that** it is obtainable by immunising a non-human animal with the peptide of sequence SEQ ID NO: 4.

13. Antibody that binds to the extracellular domain of RAMP3, **characterised in that** it is obtainable by immunising a non-human animal with the peptide of sequence SEQ ID NO: 6.

14. Method for obtaining an antibody that binds to an extracellular domain of CRLR, **characterised in that** it comprises a step of immunising a non-human animal with the peptide of sequence SEQ ID NO: 1.

15. Method for obtaining an antibody that binds to the extracellular domain of RAMP2, **characterised in that** it comprises a step of immunising a non-human animal with the peptide of sequence SEQ ID NO: 4.

16. Method for obtaining an antibody that binds to the extracellular domain of RAMP3, **characterised in that** it comprises a step of immunising a non-human animal with the peptide of sequence SEQ ID NO: 6.

17. Method for obtaining a mixture of three antibodies that bind to the peptides of sequences SEQ ID NO: 2, 3 and 5, as defined in any one of claims 1, 4 and 5, comprising:
- a step of immunising a non-human animal with the peptide of sequence SEQ ID NO: 2,
- a step of immunising a non-human animal with the peptide of sequence SEQ ID NO: 3, and
- a step of immunising a non-human animal with the peptide of sequence SEQ ID NO: 5.


### SEQUENCE LISTING

<110> UNIVERSITE AIX MARSEILLE 2 / UNIVERSITE DE LA MEDITERRANEE
   INSTITUT NATIONAL DE LA SANTE ET DE LA RECHERCHE MEDICALE
   (INSERM)
   ASSISTANCE PUBLIQUE - HOPITAUX DE MARSEILLE
   OUAFIK, L'Houcine
   MABROUK, Kamel
   KAAFARANY, Itidal
   MARTIN, Pierre-Marie
<120> ANTICORPS SE LIANT AUX RECEPTEURS DE L'ADRENOMEDULLINE ET LEURS UTILISATIONS COMME MEDICAMENT
<130> F2159PCT1
<160> 6
<170> PatentIn version 3.3
<210> 1
   <211> 25
   <212> PRT
   <213> Artificial
<220>
   <223> Peptide dérivé du fragment S6-K30 de la protéine hCRLR
<400> 1
   Ser Pro Glu Asp Ser Ile Gln Leu Gly Val Thr Arg Asn Lys Ile Met
   1 5 10 15
   Thr Ala Gln Tyr Glu Ala Tyr Gln Lys
   20 25
<210> 2
   <211> 31
   <212> PRT
   <213> Artificial
<220>
   <223> Peptide dérivé du fragment P68-R98 de la protéine hCRLR
<400> 2
   Pro Asp Tyr Phe Gln Asp Phe Asp Pro Ser Glu Lys Val Thr Lys Ile
   1 5 10 15
   Ala Asp Gln Asp Gly Asn Trp Phe Arg His Pro Ala Ser Asn Arg
   20 25 30
<210> 3
   <211> 23
   <212> PRT
   <213> Artificial
<220>
   <223> Peptide dérivé du fragment K59-K81 de la protéine hRAMP2
<400> 3
   Lys Asn Tyr Glu Thr Ala Val Gln Phe Ala Trp Asn His Tyr Lys Asp
   1 5 10 15
   Gln Met Asp Pro Ile Glu Lys
   20
<210> 4
   <211> 28
   <212> PRT
   <213> Artificial
<220>
   <223> Peptide dérivé du fragment R91-R118 de la protéine hRAMP2
<400> 4
   Arg Pro Tyr Ser Thr Leu Arg Asp Ala Leu Glu His Phe Ala Glu Leu
   1 5 10 15
   Phe Asp Leu Gly Phe Pro Asn Pro Leu Ala Glu Arg
   20 25
<210> 5
   <211> 22
   <212> PRT
   <213> Artificial
<220>
   <223> Peptide dérivé du fragment L34-K55 de la protéine hRAMP3
<400> 5
   Leu Glu Arg Leu Pro Leu Ala Gly Lys Ala Phe Ala Asp Met Met Gly
   1               5                   10                  15
   Lys Val Asp Val Trp Lys
               20
<210> 6
   <211> 22
   <212> PRT
   <213> Artificial
<220>
   <223> Peptide dérivé du fragment G91-E112 de la protéine hRAMP3
<400> 6
   Gly Phe Ile Thr Gly Ile His Arg Gln Phe Phe Ser Asn Ala Thr Val
   1               5                   10                  15
   Asp Arg Val His Leu Glu
               20